# EUROPEAN PATENT APPLICATION

(11) **EP 1 779 721 A1**
(43) Date of publication of application: **02.05.2007**
(21) Application number: 06076946.0
(22) Date of filing: 27.10.2006
(51) Int. Cl.: A01H 5/02

(54) **Gerbera with multi-petalled and globular shaped inflorescences**

(30) Priority: 28.10.2005 US 730857 P
(71) Applicant: Terra Nigra Holdings B.V., 1433 AP Kudelstaart (NL)
(72) Inventor: Boelage, P.E., 1421 KT Uithoorn (NL); Stravers, L.J.M., 1433 NG Kudelstaart (NL); Van Os, D.P.M., 3602 AT Maarssen (NL)
(74) Representative: Dohmen, Johannes Maria Gerardus

(57) **Abstract**

New, distinct and stable *Gerbera jamesonii* cultivars with multi-petalled and globular-shaped inflorescences are disclosed. The new *Gerbera jamesonii* cultivars produce one or more inflorescences with i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence.. Multi-petalled-flowering and globular-shaped *Gerbera jamesonii* cultivars are disclosed in which substantially all the inflorescences produce i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence. The multi-petalled-type and globular-shaped inflorescence characteristic has been successfully bred into single-type (at least 50, to about 75, full or partial outer petals (ray florets) per inflorescence) and semi-double-type (at least 130, to about 170, full or partial outer petals (ray florets) per inflorescence) and double-type (at least 250, to about 270, full or partial outer petals (ray florets) per inflorescence) and double-multi-type (at least 270, to about 320, full or partial outer petals (ray florets) per inflorescence) *Gerbera* cultivars. The multi-petalled-type and globular-shaped inflorescence characteristic has been combined with many desirable *Gerbera jamesonii* traits, such as inflorescence color, inflorescence quality, stem quality, inflorescence productivity and vase life. Methods for the breeding of the multi-petalled-type and globular shaped inflorescence characteristic into diverse single-type or semi-double-type or double-type or double-multi-type *Gerbera jamesonii* genetic backgrounds, as well as, methods for increasing the degree of multi-petalledness per inflorescence or plant are disclosed.

## Description

### CROSS REFERENCE TO RELATED PATENT APPLICATIONS

This application claims priority from United States Provisional Application 60/730,857 filed October 28, 2005, incorporated herein by reference in its entirety.

### BACKGROUND OF THE INVENTION

### 1. Field of the Invention

The present invention relates to new, distinct and stable cultivars of multi-petalled-type and globular-shaped *Gerbera jamesonii* plants that produce one or more inflorescences with i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence. A multi-petalled-type and globular-shaped *Gerbera jamesonii* cultivar of the instant invention produces i) at least 320, 330, 340, 350, 360, 370, 380, 390, 400, 410, 420, 430, 440, 450, 460, 470, 480, 490, 500, 510, 520, 530, 540 550, any integer between 320 and 550 or more than 550 full or partial outer petals (ray florets) per inflorescence, ii) at least 150, 160, 170, 180, 190, 200, 210, 220, 230, 240, 250, 260, 270, 280, 290, 300, any integer between 150 and 300, or more than 300 full or partial inner petals (disc florets) per inflorescence, and iii) at least 520,530, 540, 550, 560, 570, 580, 590, 600, 610, 620, 630, 640, 650, 660, 670, 680, 690, 700, 710, 720, 730, 740, 750, 760, 770, 780, 790, 800, any integer between 520 and 800, or more than 800 full or partial total petals (total florets) per inflorescence.

The present invention relates to the multi-petalled-type and globular-shaped inflorescence trait which has been successfully introgressed from single-type (at least 50, to about 75, full or partial outer petals per inflorescence) and semi-double-type (at least 130, to about 170, full or partial outer petals per inflorescence) and double-type (at least 250, to about 270, full or partial outer petals per inflorescence) and double-multi-type (at least 270, to about 320, full or partial outer petals per inflorescence) *Gerbera jamesonii* cultivars into other single-type, semi-double-type, double-type and double-multi-type *Gerbera jamesonii* plants, as well as, methods for increasing the degree of multi-petalledness per inflorescence or per plant. The multi-petalled-type and globular-shaped inflorescence characteristic has also been combined with many desirable *Gerbera jamesonii* traits, such as inflorescence color, inflorescence quality, stem quality, inflorescence productivity and vase life.

### 2. Background

The present invention relates generally to the field of ornamental *Gerbera* plants. The genus of *Gerbera* belongs to the *Asteraceae* family. There are more than 40 different species of *Gerbera,* of which the species are distributed from Africa into Madagascar into tropical Asia and into South America.

*Gerbera* is an important crop in ornamental horticulture worldwide. Through hybridization, *Gerbera* species are phenotypically diverse and offer a wide range of inflorescence colors including white, red, orange, pink, purple and yellow. Since *Gerbera* inflorescences have a long vase life, *Gerbera* inflorescences are widely used in the commercial horticultural industry. A *Gerbera* cultivar can be grown as a cut inflorescence, as well as, a potting plant.

The current invention is mainly suitable for cut flower production, though some cultivars can also be used as potting plants. The present invention allows the *Gerbera* market to expand as a completely new type of *Gerbera* inflorescence is produced. The breeding of multi-petalled-type and globular-shaped *Gerbera jamesonii* cultivars, which produce one or more inflorescences with i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, offers a unique, new *Gerbera* inflorescence form that can be combined with different *Gerbera* flowering-type species, thereby expanding the range of phenotypic characteristics available in this popular horticultural plant.

### SUMMARY OF THE INVENTION

An object of the present invention is to provide *Gerbera jamesonii* cultivars which produce one or more multi-petalled-type and globular-shaped inflorescences with at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence.

An object of the present invention is to provide *Gerbera jamesonii* cultivars wherein substantially all the flowers produced by said plant are multi-petalled and globular shaped with at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence.

Another object of the present invention is to provide *Gerbera jamesonii* cultivars wherein a multi-petalled-type and globular-shaped inflorescence has i) at least 320, 330, 340, 350, 360, 370, 380, 390, 400, 410, 420, 430, 440, 450, 460, 470, 480, 490, 500, 510, 520, 530, 540 550, any integer between 320 and 550 or more than 550 full or partial outer petals (ray florets) per inflorescence, ii) at least 150, 160, 170, 180, 190, 200, 210, 220, 230, 240, 250, 260, 270, 280, 290, 300, any integer between 150 and 300, or more than 300 full or partial inner petals (disc florets) per inflorescence, and iii) at least 520,530, 540, 550, 560, 570, 580, 590, 600, 610, 620, 630, 640, 650, 660, 670, 680, 690, 700, 710, 720, 730, 740, 750, 760, 770, 780, 790, 800, any integer between 520 and 800, or more than 800 full or partial total petals (total florets) per inflorescence.

Another object of the present invention is to provide methods for breeding of the multi-petalled type trait into diverse single-type, semi-double-type, double-type and double-multi-type *Gerbera jamesonii* genetic backgrounds.

Another object of the invention is to provide a method of breeding multi-petalled-type and globular-shaped *Gerbera jamesonii* plants that produce one or more flowers having i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, comprising the steps of (a) crossing a first single-type plant having the multi-petalled and globular shape trait in its genetic background, either as the male or female parent to (i) a semi-double-type plant; (ii) a double-type plant; (iii) a double-multi-type plant; or (iv) a second single-type plant having the multi-petalled and globular shape trait in its genetic background; (b) selecting F1 progeny that are single-type, semi-double-type, double-type or double-multi-type; (c) crossing said F1 progeny that are single-type, semi-double-type, double-type or double-multi-type to (i) a second semi-double-type plant; (ii) a second double-type plant; (iii) a second double-multi-type plant; (iv) a third single-type plant having the multi-petalled and globular shape trait in its genetic background; and (d) selecting multi-petalled and globular shaped flowering progeny. The first, second and third single-type plants, or first and second semi-double-type, double-type or double-multi-type plants are the same or different cultivars.

Another object of the invention is to provide a method of breeding a multi-petalled-type and globular-shaped *Gerbera jamesonii* plant that produces one or more inflorescences with i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, comprising the steps of (a) crossing a first semi-double-type, either as the male or female parent to (i) a second semi-double-type plant; (ii) a double-type plant; (iii) a double-multi-type plant; (iv) a single-type plant having the multi-petalled and globular shape trait in its genetic background; or (v) a single-type plant with no known multi-petalled and globular shape trait in its genetic background; (b) selecting F1 progeny that are single-type, semi-double-type, double-type or double-multi-type; (c) crossing said F1 progeny that are single-type, semi-double-type, double-type or double-multi-type to (i) a second double-type plant; (ii) a second double-multi-type plant; (iii) a third semi-double-type plant; (iv) a second single-type plant having the multi-petalled and globular shape trait in its genetic background; or (v) a second single-type plant with no known multi-petalled and globular shape trait in its genetic background; and (d) selecting multi-petalled and globular shaped flowering progeny. The first and second single-type, double-type or double-multi-type plants, or first, second and third semi-double-type plants are same or different cultivars.

Another object of the invention is to provide a method of breeding a multi-petalled-type and globular shaped *Gerbera jamesonii* plant that produce one or more inflorescences with at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, comprising the steps of (a) crossing a first double-type plant, either as the male or female parent to (i) a semi-double-type plant; (ii) a double-multi-type plant; (iii) a second double-type plant; (iv) a single-type plant having the multi-petalled and globular shape trait in its genetic background; or (iv) a single-type plant with no known multi-petalled and globular shape trait in its genetic background; (b) selecting F1 progeny that are single-type, semi-double-type, double-type or double-multi-type; (c) crossing said F1 progeny that are single-type, semi-double-type, double-type or double-multi-type to (i) a third double-type plant; (ii) a second semi-double-type plant; (iii) a second double-multi-type plant; (iv) a second single-type plant having the multi-petalled and globular shaped trait in its genetic background; or (v) a second single-type plant with no known multi-petalled and globular shaped trait in its genetic background; and (d) selecting multi-petalled-type and globular shaped flowering progeny. The first, second and third double-type plants, or first and second single-type, semi-double-type or double-multi-type plants are the same or different cultivars.

Another object of the invention is to provide a method of breeding a multi-petalled-type and globular shaped *Gerbera jamesonii* plants that produce one or more inflorescences with at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, comprising the steps of (a) crossing a first double-multi-type plant, either as the male or female parent to (i) a semi-double-type plant; (ii) a double-type plant; (iii) a single-type plant having the multi-petalled and globular shape trait in its genetic background; (iv) a single-type plant with no known multi-petalled and globular shape trait in its genetic background; or (v) a second double-multi-type plant; (b) selecting F1 progeny that are single-type, semi-double-type, double-type or double-multi-type; (c) crossing said F1 progeny that are single-type, semi-double-type, double-type or double-multi-type to (i) a second semi-double-type plant; (ii) a second double-type plant; (iii) a second single-type plant having the multi-petalled and globular shaped trait in its genetic background; (iv) a second single-type plant with no known multi-petalled and globular shaped trait in its genetic background; or (v) a third double-multi-type plant; and (d) selecting multi-petalled-type and globular shaped flowering progeny. The first, second and third double-multi-type plants, or first and second single-type, semi-double-type or double-type plants are the same or different cultivars.

Another object of the invention is to provide a method of breeding a multi-petalled-type and globular shaped *Gerbera jamesonii* plants that produce one or more inflorescences with at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, comprising the steps of (a) crossing a first multi-petalled-type and globular shaped plant, either as the male or female parent to (i) a semi-double-type plant; (ii) a double-type plant; (iii) a double-multi-type plant; (iv) a single-type plant having the multi-petalled and globular shape trait in its genetic background; (v) a single-type plant with no known multi-petalled and globular shape trait in its genetic background; or (vi) a second multi-petalled-type and globular shaped plant; (b) selecting from F1 (i) multi-petalled and globular-shaped flowering progeny; or (ii) semi-double-type, double-type or double-multi-type progeny; (c) crossing said F1 progeny that are semi-double-type, double-type or double-multi-type to (i) a second semi-double-type plant; (ii) a second double-type plant; (iii) a second double-multi-type plant; (iv) a second single-type plant having the multi-petalled and globular shaped trait in its genetic background; or (v) a second single-type plant with no known multi-petalled and globular shaped trait in its genetic background; or (vi) a third multi-petalled-type; and (d) selecting multi-petalled-type and globular shaped flowering progeny. The first, second and third multi-petalled-type plants, or first and second single-type, semi-double-type, double-type or double-multi-type plants are the same or different cultivars.

Another object of the present invention is to provide a *Gerbera jamesonii* plant, with one or more multi-petalled-type and globular-shaped inflorescences with i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, produced by one of the methods according to claims 6, 8, 10, 12 or 14.

Another object of the present invention is to provide a *Gerbera jamesonii* plant according to claim 16, wherein said plant is selected from the group consisting of the *Gerbera jamesonii* cultivars 'Tersunrutas', 'Tersram', 'Tersunev', 'Ternoom' and 'Terlob'.

### BRIEF DESCRIPTION OF THE DRAWINGS

The patent or application file contains at least one drawing executed in color. Copies of this patent or patent application publication with color drawings will be provided by the Office upon request and payment of the necessary fees.

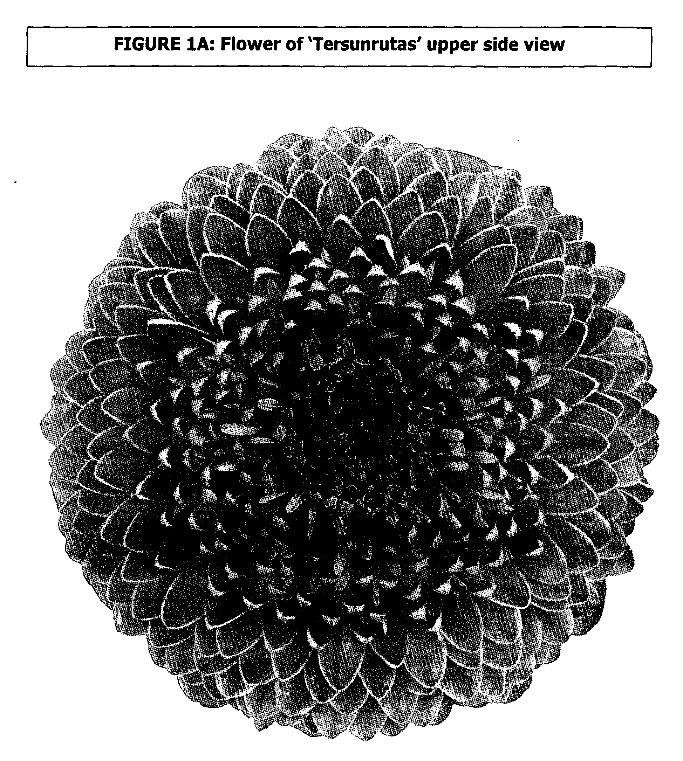

FIGURE 1A. A top perspective view of a typical inflorescence of *Gerbera jamesonii* cultivar 'Tersunrutas'.

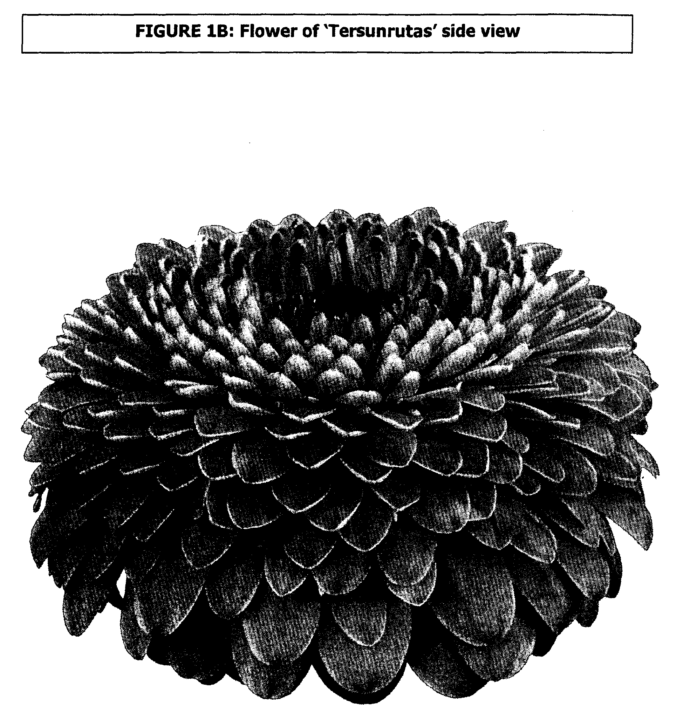

FIGURE 1B. A side perspective view of a typical inflorescence of *Gerbera jamesonii* cultivar 'Tersunrutas'.

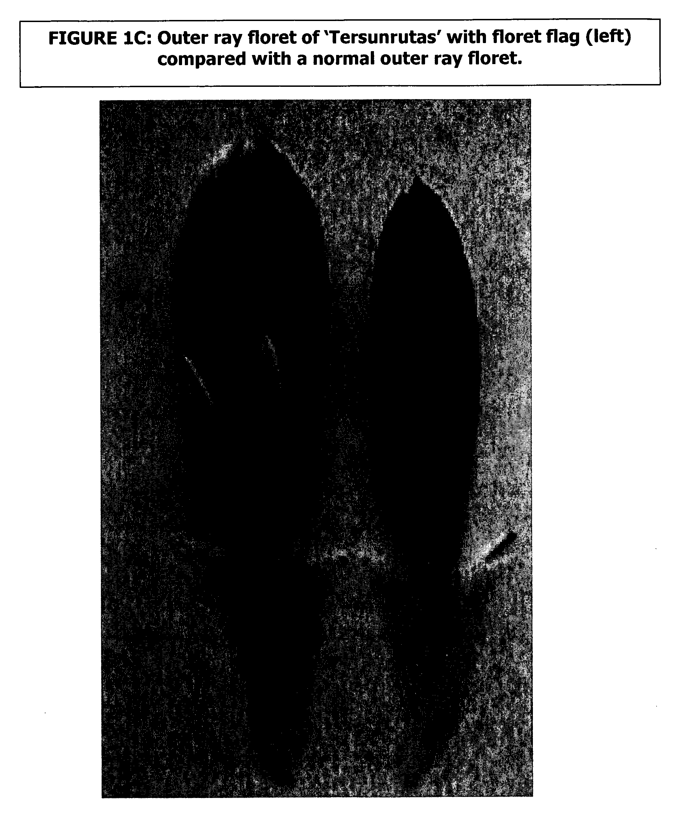

FIGURE 1C. A close-up view of a typical outer ray floret compared to a typical ray floret flag of a inflorescence of *Gerbera jamesonii* cultivar 'Tersunrutas'.

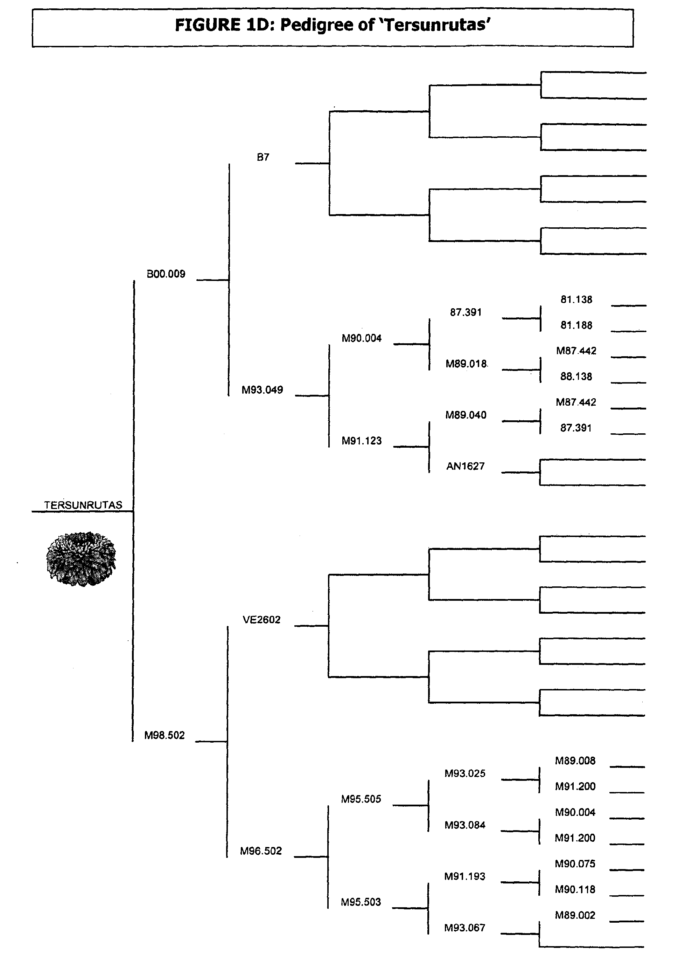

FIGURE 1D. Genealogy of multi-petalled, globular shaped *Gerbera jamesonii* cultivar 'Tersunrutas'.

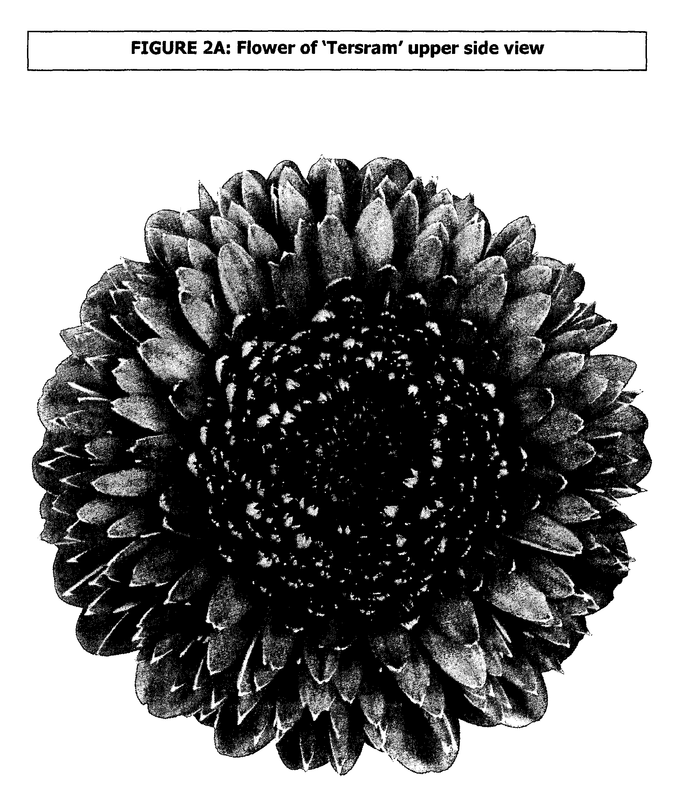

FIGURE 2A. A top perspective view of a typical inflorescence of *Gerbera jamesonii* cultivar 'Tersram'.

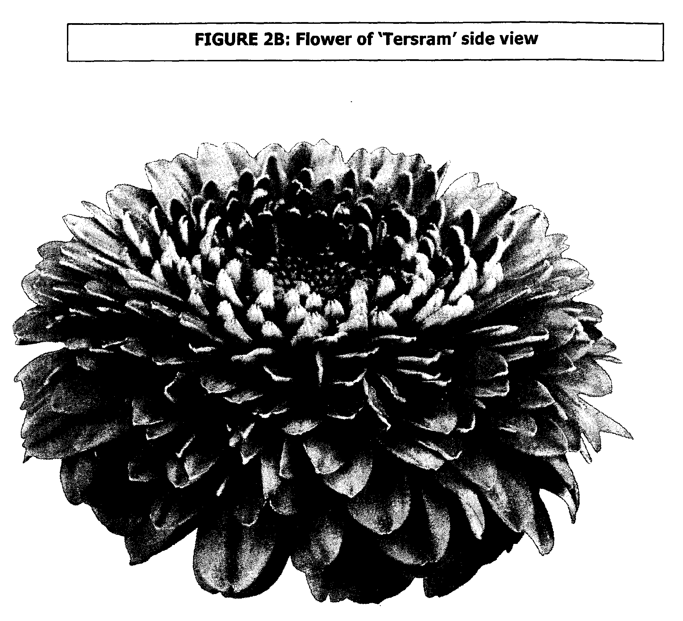

FIGURE 2B. A side perspective view of a typical inflorescence of *Gerbera jamesonii* cultivar 'Tersram'.

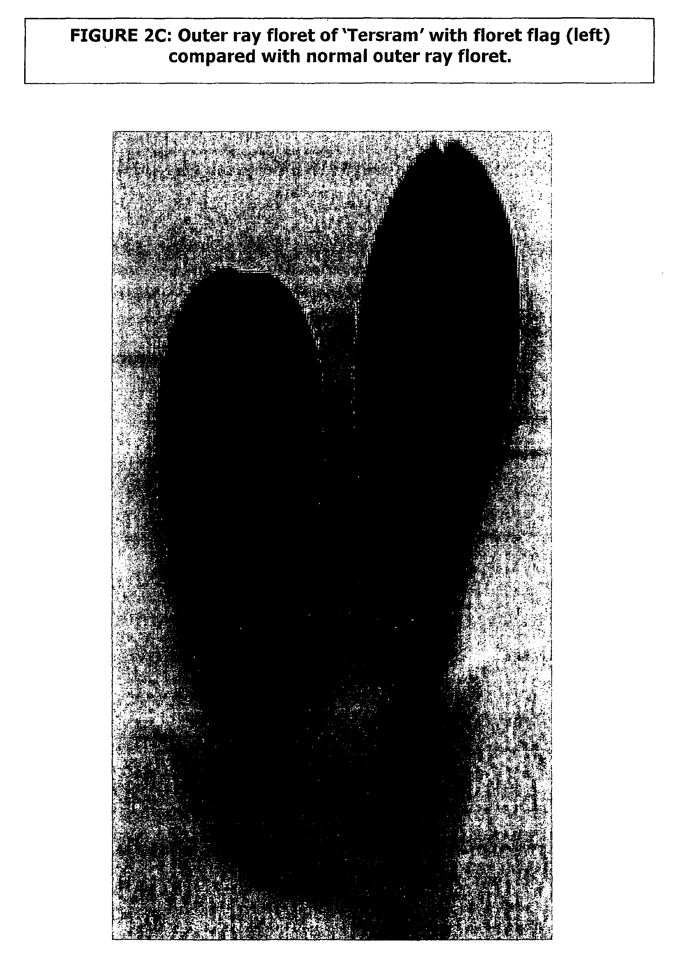

FIGURE 2C. A close-up view of a typical outer ray floret compared to a typical ray floret flag of a inflorescence of *Gerbera jamesonii* cultivar 'Tersram'.

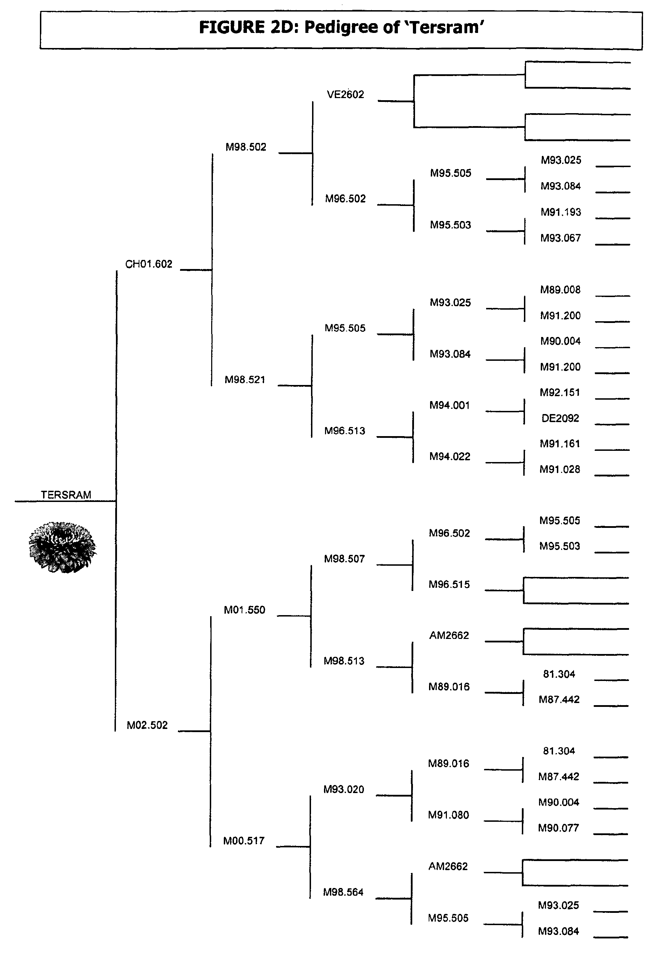

FIGURE 2D. Genealogy of multi-petalled, globular shaped *Gerbera jamesonii* cultivar 'Tersram'.

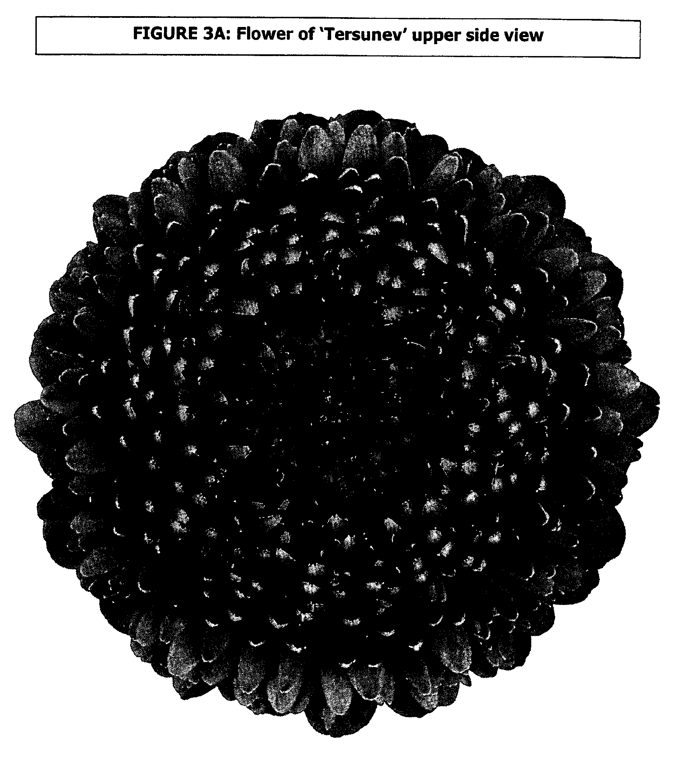

FIGURE 3A. A top perspective view of a typical inflorescence of *Gerbera jamesonii* cultivar 'Tersunev'.

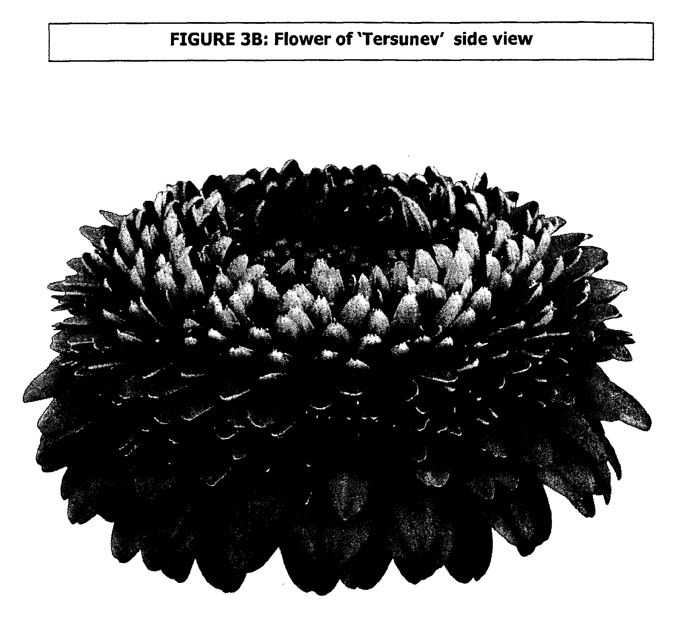

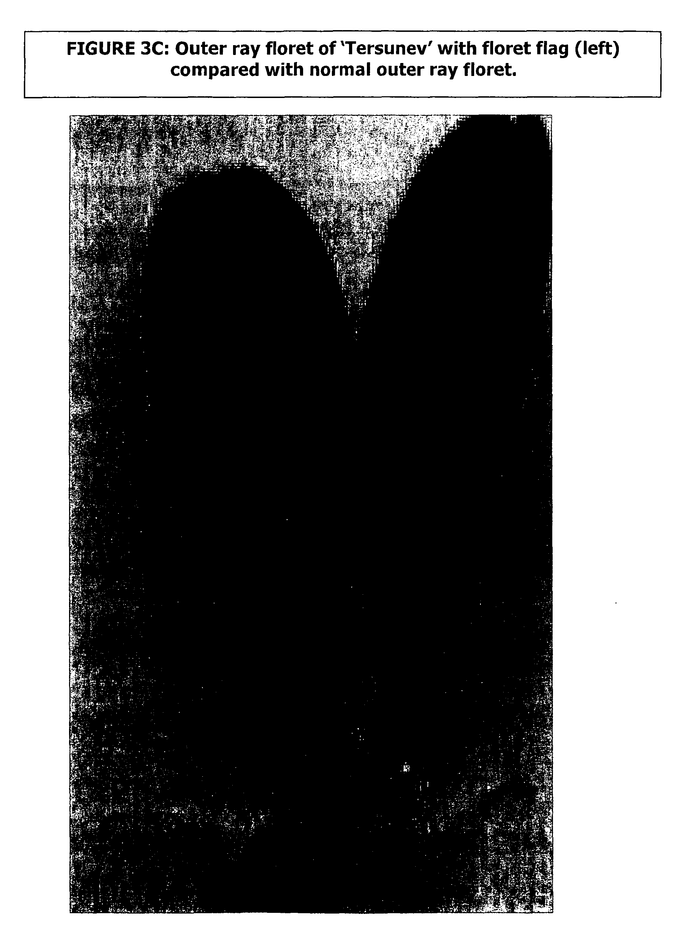

FIGURE 3B. A side perspective view of a typical inflorescence of *Gerbera jamesonii* cultivar 'Tersunev'.

FIGURE 3C. A close-up view of a typical outer ray floret compared to a typical ray floret flag of a inflorescence of *Gerbera jamesonii* cultivar 'Tersunev'.

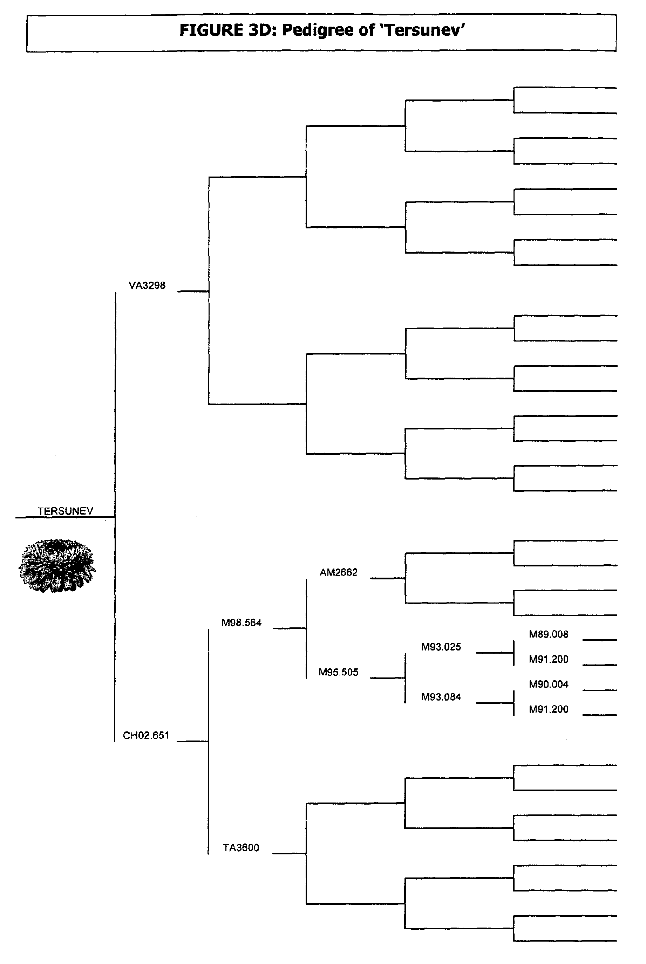

FIGURE 3D. Genealogy of multi-petalled, globular shaped *Gerbera jamesonii* cultivar 'Tersunev'.

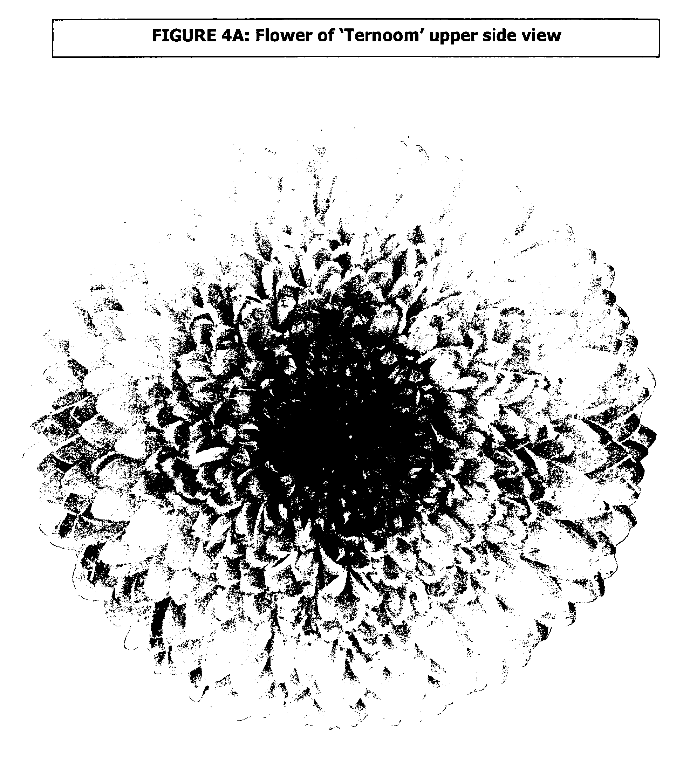

FIGURE 4A. A top perspective view of a typical inflorescence of *Gerbera jamesonii* cultivar 'Ternoom'.

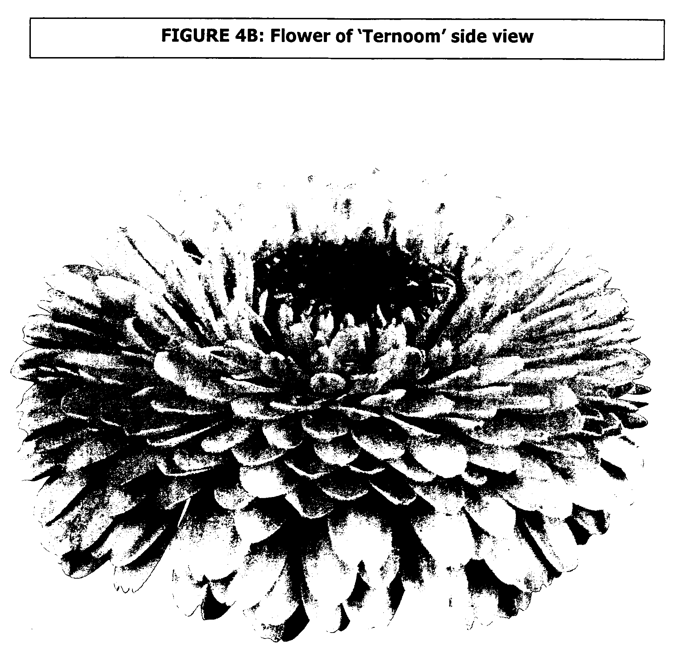

FIGURE 4B. A side perspective view of a typical inflorescence of *Gerbera jamesonii* cultivar 'Ternoom'.

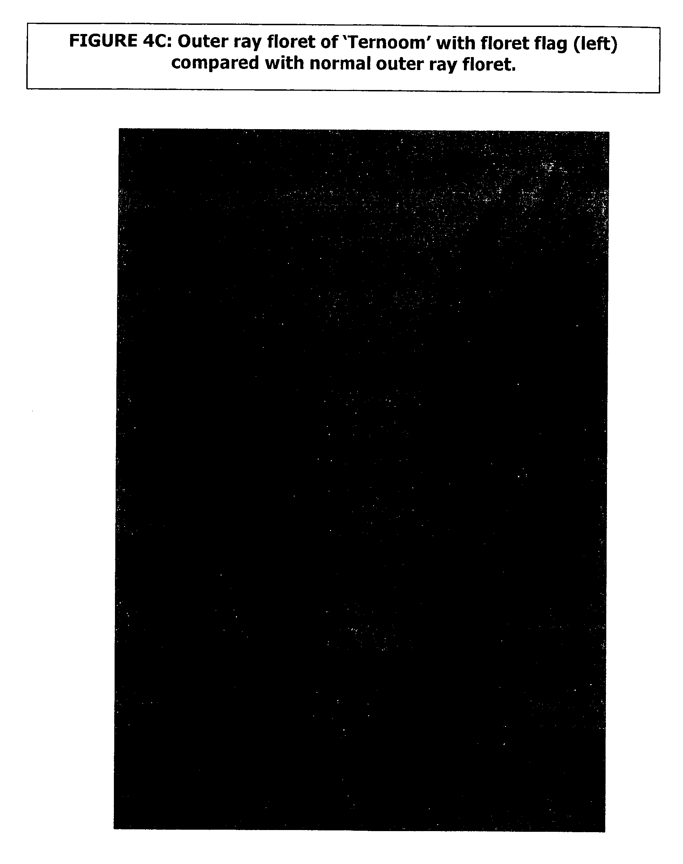

FIGURE 4C. A close-up view of a typical outer ray floret compared to a typical ray floret flag of a inflorescence of *Gerbera jamesonii* cultivar 'Ternoom'.

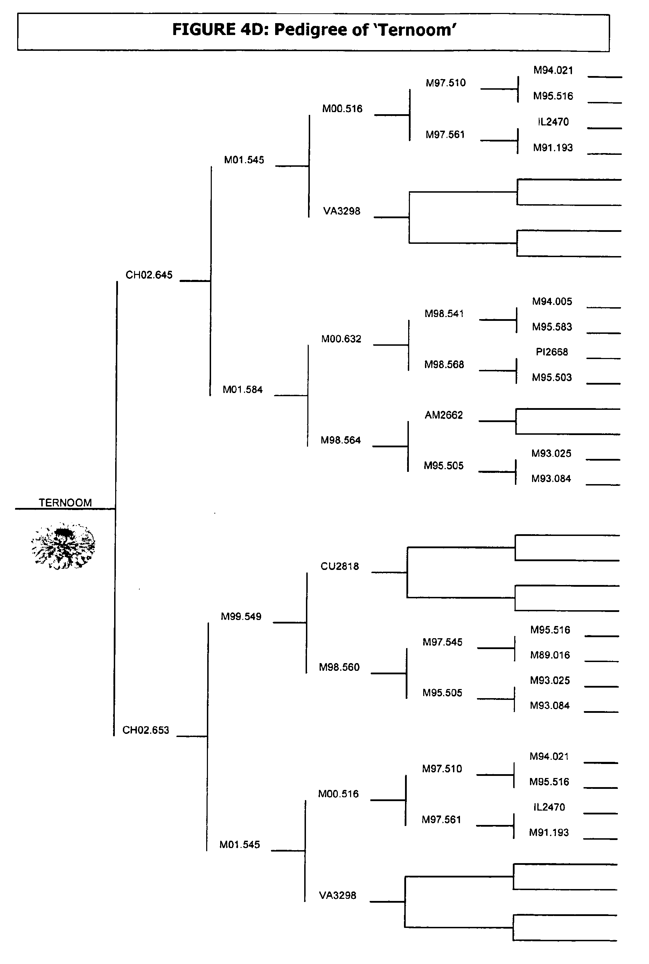

FIGURE 4D. Genealogy of multi-petalled, globular shaped *Gerbera jamesonii* cultivar 'Ternoom'.

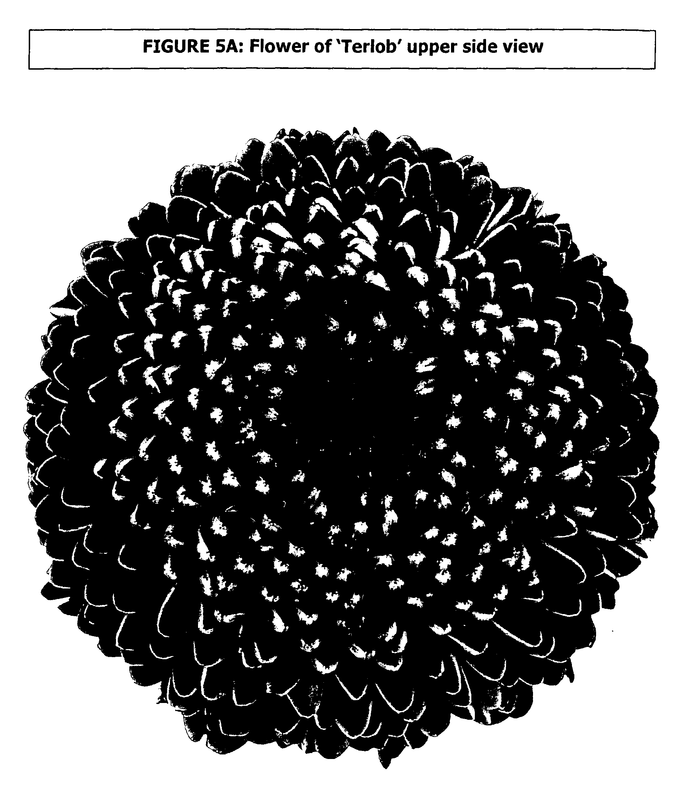

FIGURE 5A. A top perspective view of a typical inflorescence of *Gerbera jamesonii* cultivar 'Terlob'.

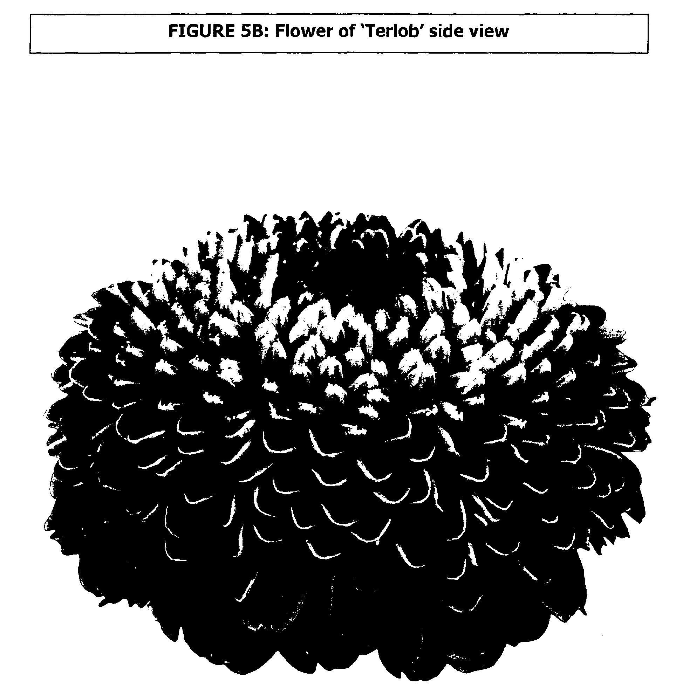

FIGURE 5B. A side perspective view of a typical inflorescence of *Gerbera jamesonii* cultivar 'Terlob'.

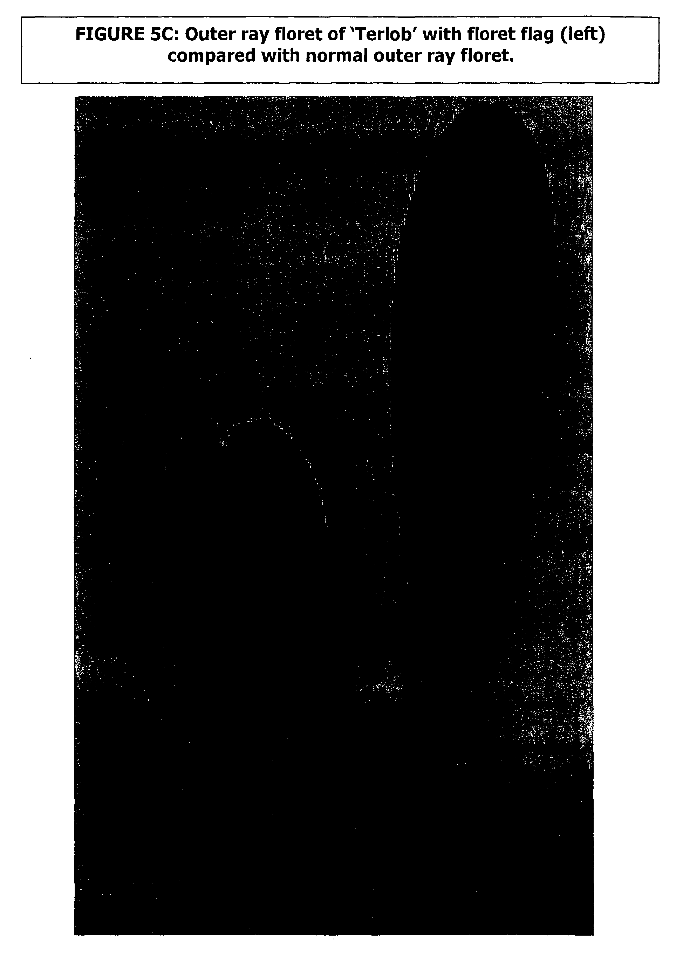

FIGURE 5C. A close-up view of a typical outer ray floret compared to a typical ray floret flag of a inflorescence of *Gerbera jamesonii* cultivar 'Terlob'.

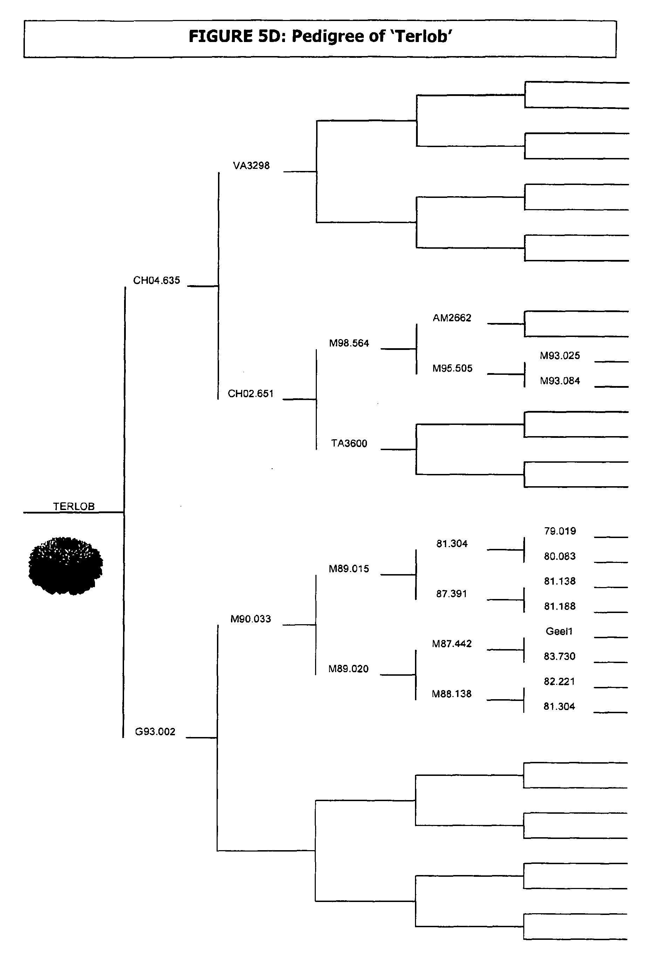

FIGURE 5D. Genealogy of multi-petalled, globular shaped *Gerbera jamesonii* cultivar 'Terlob'.

### DETAILED DESCRIPTION OF THE INVENTION

### 1. Definitions

In the description and tables which follow, a number of terms are used. In order to provide a clear and consistent understanding of the present invention, the following definitions are provided:

A *cultivar* or *variety,* is a group of similar plants which belong to the same species and which by structural features and performance may be distinguished from other varieties within the same species. Two essential characteristics of a variety are identity and reproducibility. Identity is necessary so that the variety may be recognized and distinguished from other varieties within the crop species. The distinguishing features may be morphological characteristics, color markings, physiological functions, disease/pest resistance/susceptibility, or performance. Most agricultural varieties are pure for those characteristics which identify the variety. Reproducibility is needed so that the characteristics by which the variety is identified will be reproduced uniformly and stably in the progeny. A variety is derived from a strain; populations which are increased from a single genotype or a mixture of genotypes are referred to as strains, experimental strains, or lines. Once a strain is identified as superior, it may be named, increased, and made available commercially as a "cultivated variety" or "cultivar." The words "variety" and "cultivar" are used interchangeably, although cultivar is commonly used in scientific literature while variety is the term used by U.S. farmers and the seed trade.

*Single* or *single-flowering* or *single-type* are each defined as a *Gerbera jamesonii* plant which produces one or more inflorescences having at least 50, to about 75, full or partial outer petals (ray florets) per inflorescence or a *Gerbera jamesonii* inflorescence which has at least 50, to about 75, full or partial outer petals (ray florets).

*Semi-double* or *semi-double-flowering* or *semi-double-type* are each defined as a *Gerbera jamesonii* plant which produces one or more inflorescences having at least 130, to about 170, full or partial outer petals (ray florets) per inflorescence or a *Gerbera jamesonii* inflorescence which has at least 130, to about 170, full or partial outer petals (ray florets).

*Double or double-flowering or double-type* are each defined as a *Gerbera jamesonii* plant which produces one or more inflorescences having at least 250, to about 270, full or partial outer petals (ray florets) per inflorescence, or a *Gerbera jamesonii* inflorescence which has at least 250, to about 270, full or partial outer petals (ray florets).

*Double-multi or double-multi-flowering or double-multi-type* are each defined as a *Gerbera jamesonii* plant which produces one or more inflorescences having at least 270, to about 320, full or partial outer petals (ray florets) per inflorescence, or a *Gerbera jamesonii* inflorescence which has at least 270, to about 320, full or partial outer petals (ray florets). All double-multi-types are half products. The multi-petalling trait does not express itself to the fullest extent until after two generations.

*Multi-petalled, multi-petalled-flowering or multi-petalled-type* are each defined as a *Gerbera jamesonii* plant which produces one or more inflorescences having at least 320, to about 550, full or partial outer petals (ray florets) per inflorescence or a *Gerbera jamesonii* inflorescence which has at least 320, to about 550, full or partial outer petals (ray florets). Preferably, the multi-petalled-type *Gerbera jamesonii* plant of the instant invention has substantially all multi-petalled-type inflorescences. The multi-petalled-type inflorescences of the instant invention have i) at least 320, 330, 340, 350, 360, 370, 380, 390, 400, 410, 420, 430, 440, 450, 460, 470, 480, 490, 500, 510, 520, 530, 540 550, any integer between 320 and 550 or more than 550 full or partial outer petals (ray florets) per inflorescence, ii) at least 150, 160, 170, 180, 190, 200, 210, 220, 230, 240, 250, 260, 270, 280, 290, 300, any integer between 150 and 300, or more than 300 full or partial inner petals (disc florets) per inflorescence, and iii) at least 520, 530, 540, 550, 560, 570, 580, 590, 600, 610, 620, 630, 640, 650, 660, 670, 680, 690, 700, 710, 720, 730, 740, 750, 760, 770, 780, 790, 800, any integer between 520 and 800, or more than 800 full or partial total petals (total florets) per inflorescence. A multi-petalled-type *Gerbera jamesonii* plant of the instant invention may produce inflorescences with a relatively uniform number of petals per inflorescence and this characteristic is stable through asexual reproduction. Alternatively, a multi-petalled-type *Gerbera jamesonii* plant of the instant invention may produce inflorescences with a wide range in the number of petals per inflorescence and this characteristic is also stable through asexual reproduction.

The *degree of multi-petalledness per inflorescence* is defined as a measure of the number of extra full or partial outer petals (ray florets) per inflorescence produced beyond the 250-270 full or partial outer petals (ray florets) typically found on double-type *Gerbera jamesonii.* The greater the degree of multi-petalledness per inflorescence, the greater the number of full or partial outer petals (ray florets) produced per inflorescence.

The *degree of multi-petalledness per plant* is defined as a measure of the number of inflorescences per *Gerbera jamesonii* plant which have at least 320 full or partial (ray florets) petals per inflorescence. The greater the degree of multi-petalledness per *Gerbera jamesonii* plant, the higher the percentage of total inflorescences produced by the *Gerbera jamesonii* plant which have at least 320 full or partial outer petals (ray florets) per inflorescence.

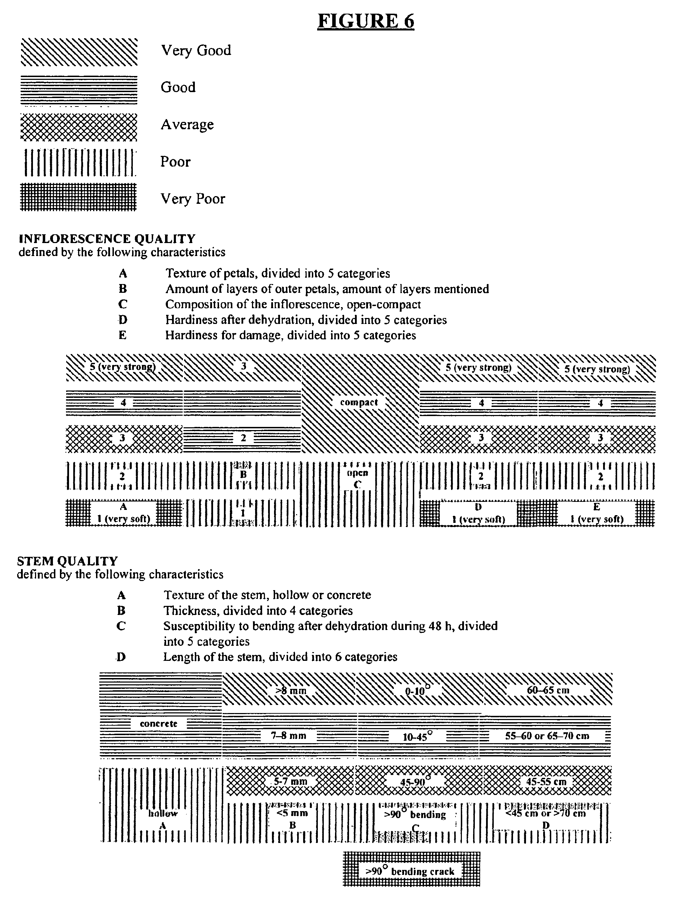

The *inflorescence quality, stem quality, inflorescence productivity and vase life per plant,* as referenced in Table 1-6 and throughout specification, is defined as a measure of the 5 ratings (very good, good, average, poor, very poor), which are further defined in Figure 6.

### INFLORESCENCE QUALITY

defined by the following characteristics
- **A**: Texture of petals, divided into 5 categories
- **B**: Amount of layers of outer petals, amount of layers mentioned
- **C**: Composition of the inflorescence, open-compact
- **D**: Hardiness after dehydration, divided into 5 categories
- **E**: Hardiness for damage, divided into 5 categories

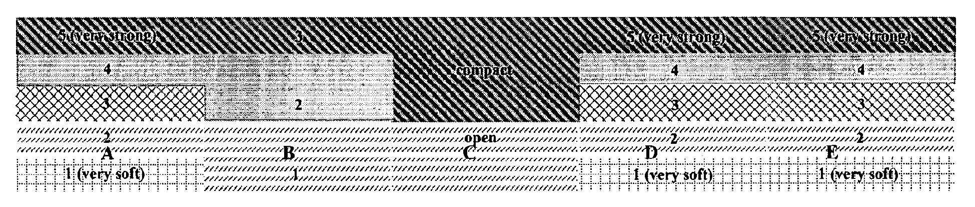

### STEM QUALITY

defined by the following characteristics
- **A**: Texture of the stem, hollow or concrete
- **B**: Thickness, divided into 4 categories
- **C**: Susceptibility to bending after dehydration during 48 h, divided into 5 categories
- **D**: Length of the stem, divided into 6 categories

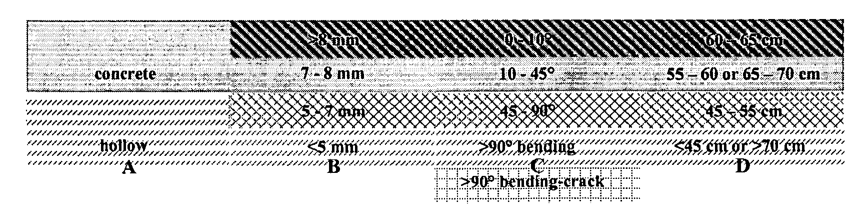

*Vase life* and *productivity per plant* are defined as:

| | **VASELIFE** | **PRODUCTION** |
|---|---|---|
| | | for flower size 7-8 cm |
| | (days) | (flowers/plant/year) |
| very good | >16 | >79 |
| good | 12-16 | 71-79 |
| average | 8-12 | 58-70 |
| poor | <8 | <58 |

*Mini-Gerbera* is a term the inventors assigned to *Gerbera jamesonii* cultivars with a flower head diameter measuring about 7.0 cm.

*Inflorescence* is defined as a type of flower in which there is more than one flower in a single structure.

*Flower Head* is defined as a composite inflorescence type which contains a dense, indeterminate mass of small, individual flowers (often called florets) that together appear to form a single flower. The compact cluster of florets are sessile or subsessile and are crowded on a compound receptacle, sometimes called a capitulum. The capitulum of an *Asteraceae*/*Compositae* can be composed of hundreds of individual flowers even though the flower head may appear to be only a single flower.

*Perianth* is defined as the part of the flower which surrounds the reproductive organs, generally the calyx (containing the sepals) and the corolla (containing the petals).

*Calyx* is defined as the outermost part of the perianth of a flower. The calyx is usually small and green but sometimes can be showy and brightly colored. The calyx of a flower is formed from the sepals (small leaves located directly under the flower) and which encloses the petals in a bud.

*Corolla* is defined as the inner part of the perianth of a flower.. If the units of the corolla are separate, they are called petals, and the corolla is said to be polypetalous.

*Petal* is defined as a unit of the corolla, which are usually colored or more or less showing. When the flower is open, the petals are located between the sepals and the flower's reproductive organs. In a flower head, the petals of a corolla arise from the same point and form a circle or circles which can be referred to as a whorl or whorls.

*Floret* is defined as a very small, individual flower in a flower head, especially when part of a dense inflorescence, such as *Composite.* Plants in the *Asteraceae*/*Compositae* family typically include one or both types of florets: ray florets and disc florets. Ray florets and disc florets of *Asteraceae*/*Compositae* plants are actinomorphic.

*Ray Floret* is defined as the "petal-like" part of a flower head which is characterized as long and strap-like (also referred to as a ligule). In a flower head of *Asteraceae*/*Compositae* plants, the outermost whorl or whorls of "petal-like" ligules are ray florets. In this invention, ray florets are sometimes referred to as "outer petals".

*Disc Floret* is defined as a small "petal-like" flower with a tubular corolla which is located in the inner whorl of a flower head of *Asteraceae*/*Compositae* plants. In this invention, disc florets are sometimes referred to as "inner petals".

*Pappus* is defined as the part of individual ray and disc florets that surrounds the base of a composite inflorescence, and which may be covered with bristle-like teeth or scales generally undetectable by the unaided eye.

### 2. Plant Selection and Breeding

General breeding methods of *Gerbera* cultivars is described in Barigozzi, C. and L. Quagliotti, 1978, "Current Research on Breeding of Gerbera", Proceedings of the Eucarpia Meeting on Carnation and Gerbera, Allasio, pp. 57-68, and Van Os, D.P.M., 1995, "Stageverslag Gerbera Veredeling, Practical Period Agriculture", University Wageningen, Dept. of Plant Breeding.

The development of multi-petalled, globular-shaped *Gerbera jamesonii* cultivars has been a gradual process involving many selections.

The first crossings in the breeding program were made in an effort to develop a *Gerbera jamesonii* cultivar with a flower head that is smaller than typical *Gerbera jamesonii* cultivars which measure up to 12.0 cm in flower head diameter. The inventors' aim was to develop smaller *Gerbera jamesonii* cultivars with a flower head diameter measuring about 7.0 cm. From the progeny of the first crosses, the inventors discovered and selected a *Gerbera jamesonii* cultivar with a flower head diameter of about 7.0 cm and called it mini-*Gerbera.* Representative mini-*Gerbera* varieties are found in U.S. Plant Patents numbers PP7,991, PP7,984, PP8,078.

Table 1 shows the most important parents used and selected based on their specific characteristics, to obtain smaller *Gerbera jamesonii* inflorescences with higher productivity and improved vase life.

**Table 1: Most important parents which have been used to invent smaller Gerbera jamesonii.**

| Designation | Flower Head General Color | Flowering Type | Flower Head Center Color | Flower Head Diameter (cm) | Inflorescence Quality | Vase Life |
|---|---|---|---|---|---|---|
| '81.139' | Pink | Semi-double | Green | 11.0-12.0 | Average | Poor |
| '81.304' | Red | Double | Green | 9.0-10.0 | Poor | Poor |
| '82.221' | Pink | Single | Green | 9.0-10.0 | Poor | Poor |
| '87.391' | Red | Semi-double | Green | 8.0-9.0 | Poor | Poor |
| '88.138' | Pink | Semi-double | Green | 8.0-9.0 | Poor | Good |

Table 2 shows the most important seedlings resulting from the crossings of Table 1, and which play an important role in the current invention.

**Table 2: Most important seedlings from crosses for smaller inflorescences.**

| Designation | Flower Head General Color | Flowering Type | Flower Head Center Color | Flower Head Diameter (cm) | Flower Quality | Petal Count | | Vase Life | Special Trait |
|---|---|---|---|---|---|---|---|---|---|
| | | | | | | Outer Petals | Inner Petals | | |
| 'M87.442' | Light Red | Single | Black | 7.0 | Average | 55-60 | | Very good | Productivity |
| 'M89.008' | Yellow | Semi-double | Black | 7.0-8.0 | Average | 52 | 108 | Very good | Plant type |
| 'M89.011' | Orange | Semi-double | Black | 6.0-7.0 | Average | 56 | 120 | Good | Short petals |
| 'M89.016' | Red | Single | Black | 7.0 | Average | 60 | | Very good | Productivity |
| 'M89.09' | Red/White | Semi-double | Green | 7.0 | Average | 55 | 88 | Good | |
| 'M89.020' | Pink | Semi-double | Black | 7.0 | Good | 52 | 80 | Very Good | Quality |
| 'M90.004' | Yellow | Single | Green | 7.0 | Average | 60 | | Good | |
| 'M90.007' | Pink | Semi-double | Green | 7.0 | Average | 53 | 97 | Good | |
| 'M90.033' | Pink Mix | Double | Black | 7.0-8.0 | Good | 87 | 124 | Good | High Petal Count |
| 'M90.077' | Lilac | Semi-double | Black | 7.0-8.0 | Poor-average | | | Good | |
| 'M90.129' | Pink | Semi-double | Black | 7.0 | Average | | | Good | |
| 'M90.149' | Pink | Semi-double | Black | 7.0 | Average | | | Average | |
| 'M91.179' | Yellow | Semi-double | Green | 7.0-8.0 | Average | 65 | | Good | Color, petal arrangement |

The inventors further recognized that some mini-*Gerbera* cultivars were also the first selections with more than the typical number of petals found among double-type flowering *Gerbera jamesonii* cultivars. The inflorescences produced by these *mini-Gerbera* cultivars comprised i) more than 270 full or partial outer petals (ray florets) per inflorescence, and ii) more than 400 full or partial total petals (total florets) per inflorescence).

Since a disadvantage of *Gerbera jamesonii* cultivars is the susceptibility to damage during transport, the inventors continued research and development to breed additional mini-*Gerbera* cultivars with increased petal count. Increasing the amount of petals per *Gerbera jamesonii* inflorescence can reduce damage during transport, and thus, would benefit the *Gerbera* horticultural industry.

Five new *Gerbera jamesonii* cultivars were selected that produce a larger number of petals than typical double-type inflorescences. In Table 3, these 5 seedlings are listed with their respective traits.

**Table 3: First seedlings coming from the breeding program expressing more than typical double-type flowering**

| Designation | Flower Head General Color | Flowering Type | Flower Head Center Color | Flower Head Diameter (cm) | inflorescence Quality | Petal Count | | Vase Life | Special Remark |
|---|---|---|---|---|---|---|---|---|---|
| | | | | | | Outer Petals | Inner Petals | | |
| 'M91.118' | Soft Pink | Double | Black | 7.0-8.0 | Average-good | | | Good | No functional pollen |
| 'G93.002' | Pink Mix | Double | Black | 7.0-8.0 | Average-good | 97 | 200 | Good | Functional pollen |
| 'G93.004' | Purple | Double | Black | 8.0 | Average | 280 | 130 | Average | Functional pollen |
| 'G93.005' | Yellow | Double | Green | 8.0 | Average | 348 | 120 | Average | No functional pollen |
| 'G93.006' | Cream | Double | Green | 7.0-8.0 | Good | 189 | 100 | Good | No functional pollen |

The extra-double seedling designated 'M91.118' was first discovered amongst the seedling population resulting from the cross of *Gerbera jamesonii* cultivars designated 'M90.129' and 'M90.149'. Both parents were characterized as semi-double-types with pink petals and a black center. The resulting seedling was characterized as a double-type with light pink petals and a black center.

Two years later, four more seedlings were selected for their very-double-flowering-type, resulting from crossings made between the bolded parents in Table 2.

As indicated in Table 3, the total petal count increased from an average of 100-120 total petals per inflorescence to an average of about 360 total petals (range from 289 to 468 total petals per inflorescence) per inflorescence. Accordingly, the inventors had successfully discovered new *Gerbera jamesonii* cultivars which exceeded the typical double-type inflorescence petal count of 250-270.

The mini-*Gerbera* cultivars with increased petal count appeared in the selection material. However, the higher petal count of the mini-*Gerbera* was not reason enough, at that time, to promote a successful introduction into the commercial horticulture market.

The development of *mini-Gerberas* continued rapidly, and this created the opportunity for the inventors to focus their efforts on also improving inflorescence quality, shape and productivity of *mini-Gerberas.* The inventors focused on discovering and selecting new, improved *Gerbera jamesonii* cultivars which produce inflorescences with smaller and stronger petals, a higher petal count, and a more compact flower head size.

In Table 4 the most important parents are listed that form the basis of the new breeding program. The parents can be divided into two main categories. One group of parents passes its characteristics regarding petal count, shape and formation. The other group of parents passes its characteristics concerning vase life, inflorescence and stem quality and productivity.

**Table 4: Important parents used in the breeding program.**

| Designation | Flower Head General Color | Flowering Type | Flower Head Center Color | Flower Head Diameter (cm) | Inflorescence Quality | Petal Count | | Vase Life | Important Characteristic(s) |
|---|---|---|---|---|---|---|---|---|---|
| | | | | | | Outer Petals | Inner Petals | | |
| 'M93.020' | Dark Yellow | Single | Black | 7.0-8.0 | Average | 53 | | Good | Productivity |
| 'M95.503' | Dark Yellow | Single | Black | 7.0 | Extremely Good | 51 | | Extremely Good | Great inflorescence and stem quality |
| 'M95.505' | Yellow/ Orange | Semi-double | Black | 7.0-8.0 | Very Good | 60 | 100 | Very Good | Petal count, quality, vase life |
| 'M95.516' | Red | Semi-double | Black | 8.0 | Very Good | 150 | | Very Good | Extremely good inflorescence quality |
| 'M96.513' | Purple Mix | Single | Black | 7.0 | Extremely Good | 55 | | Extremely Good | Great inflorescence and stem quality |
| 'M97.510' | Red | Single | Green | 7.0 | Extremely Good | 60 | | Extremely Good | Great inflorescence and stem quality |
| 'M98.507' | Yellow | Single | Black | 7.0 | Good | 66 | | Good | Color, quality and stem length |
| 'M00.516' | Cream/ Peach | Semi-double | Green | 7.0-8.0 | Very Good | 56 | 120 | Very Good | High petal county, quality |
| 'M00.632' | Orange | Single | Black | 7.0 | Extremely Good | 58 | | Extremely Good | Great inflorescence and stem quality |

New seedlings were selected by the inventors based on the characteristic of producing *more than typical* double-type flowering. The breeding program intensified and focused specifically on crossing *Gerbera jamesonii* cultivars which produced inflorescences with increased petal count, without a complete loss of fertility. Additional parental characteristics considered by the inventors were the shape of the petals, the arrangement of the petals in the inflorescence, the division between male and female florets and the general quality of the inflorescence. This resulting generation showed to be fertile.

The parents listed in Table 5 were used in the breeding program. All parents complied with at least one of the above listed required and important characteristics.

**Table 5: Parents used in breeding program.**

| Designation | Flower Head General Color | Flowering type | Flower Head Center Color | Flower Head Diameter (cm) | Inflorescence Quality | Petal count | | Vase Life | Important Characteristic(s) |
|---|---|---|---|---|---|---|---|---|---|
| | | | | | | Outer Petals | Inner Petal | | |
| 'M90.033' | Pink Mix | Double | Black | 7.0-8.0 | Good | 74 | 184 | Good | High petal count |
| 'M96.502' | Orange | Semi-double | Black | 7.0 | Very Good | 63 | 98 | Very Good | Very good quality, lower productivity |
| 'M98.502' | Yellow/orange | Semi-double | Black | 8.0 | Very Good | 90 | 180 | Very Good | Inflorescence quality, petal count, production |
| 'M98.521' | Red | Semi-double | Black | 7.0-8.0 | Very Good | 54 | 162 | Very Good | Color, quality and productivity |
| 'M98.564' | Orange | Semi-double | Black | 7.0 | Very Good | 66 | 134 | Very Good | Stem length, productivity |
| 'M99.549' | Cream | Semi-double | Black | 7.0-8.0 | Good | 58 | 123 | Good | High production |
| 'M00.517' | Orange | Semi-double | Black | 7.0 | Very Good | 111 | 120 | Very Good | Extremely good quality, different petal arrangement |
| 'M01.545' | Pink | Semi-double | Green | 7.0-8.0 | Very Good | 73 | 114 | Very Good | Productivity |
| 'M01.584' | Orange | Semi-double | Black | 7.0 | Very Good | 61 | 104 | Very Good | Inflorescence quality |
| 'TA3600' | orange | Double | black | 7 cm | Good | 80 | 178 | good | Petal count |
| 'VA3298' | cream | Single | green | 7-8 cm | Good | 68 | | good | Inflorescence shape |
| 'VE2602' | cream/pink | semi-double | black | 11-12 cm | Good | 45 | 124 | average | Color |

Selected seedlings were put into a tissue culture laboratory, so that large scale production trials could be carried out.

An attractive multi-petalled and globular shaped *Gerbera jamesonii* cultivar was developed and designated 'Tersunrutas.' The 'Tersunrutas' seedling was selected in 2002, introduced into the laboratory in 2002, propagated in 2003, and thoroughly tested in 2003 and 2004. This cultivar is included in this description of the invention as Example 2. In 2004 and 2005, four additional multi-petalled and globular shaped *Gerbera jamesonii* cultivars were discovered in different colors ('Tersram', 'Tersunev', 'Ternoom' and 'Terlob'). These cultivars are included in this description of the invention as Examples 3-6. All of these new *Gerbera jamesonii* cultivars comply with the initially set standards of flower head diameter size of about 6.0 to 8.0 cm, increased petal count (with at least 320, to about 550, full or partial outer petals (ray florets) per inflorescence), good vase life (minimum of 12 days), minimum stem length of 50 cm, open plant types, and sufficient productivity (≥50 inflorescences/plant/year).

The results of the newly invented group of multi-petalled and globular shaped *Gerbera jamesonii* cultivars are listed in Table 6.

**Table 6: Summary of results of the breeding program for multi-petalled and globular shaped Gerbera jamesonii varieties.**

| Designation | Flower Head General Color | Flowering Type | Flower Head Center Color | Flower Head Diameter (cm) | Inflorescence Quality | Petal Count | | Vase life |
|---|---|---|---|---|---|---|---|---|
| | | | | | | Outer Petals | Inner Petals | |
| 'Tersunrutas' | Pink | Multi-petalled | Green | 7.5-8.0 | Very Good | 472 | 250-280 | Good |
| 'Tersram' | Red | Multi-petalled | Dark | 7.5-8.0 | Very Good | 419 | 190-220 | Very Good |
| 'Tersunev' | Purple | Multi-petalled | Dark | 6.5-7.0 | Very Good | 335 | 185-210 | Very Good |
| 'Ternoom' | Cream | Multi-petalled | Dark | 7.0-7.5 | Good | 492 | 195-220 | Average-good |
| 'Terlob' | Purple | Multi-petalled | Dark | 6.0-6.5 | Very Good | 492 | 165-195 | Good |

Seeds from *Gerbera jamesonii* selection containing the multi-petalled and globular shaped trait were deposited in the American Type Culture Collection (ATCC), P.O. Box 1549, Manassas, VA 20108, U.S.A., and accorded ATCC deposit accession number PTA-7892. 625 seeds were deposited with the ATCC on September 25, 2006. The deposited seeds produce *Gerbera jamesonii* plants that can be crossed, either as the male or female parent, to diverse genetic backgrounds of single-type, semi-double-type, double-type, or double-multi-type *Gerbera jamesonii* species to reproducibly and predictably produce new multi-petalled and globular-shaped selections according to the methods described herein.

### 4. Plant Growth Conditions

Seedlings were grown in course-grade coco peat mixture in pots with a diameter of 19.0 cm. No growth retardants were used, and only chemicals typical to keeping *Gerbera* plant material free of pests and diseases was used.

Meristem plants were grown in Rockwool slabs, with 5 plants per slab, in a greenhouse with a daily average temperature of 18°C-20°C, during the day the temperature averages about 20°C, and during the night the temperature averages about 16°C. The plants were grown under natural light conditions supplemented with artificial light. When natural light is less than 250 W/m², artificial light is used, increasing the light intensity to 750 W/m². When natural light intensities exceed 750 W/m², screens will be closed or the greenhouse will be whitewashed if the high intensity is expected to last long. Plants were grown in a peat-based soil mix and were watered with a solution i) containing 0.8 µmol/l ammonium, 1.0 µmol/l potassium, 0.9 µmol/l natrium, 2.0 µmol/l calcium, 0.5 µmol/l magnesium, less than 0.2 µmol/l silicium, 1.0 µmol/l nitrate, 0.5 µmol/l chloride, 1.1 µmol/l sulphate, less than 0.2 µmol/l bicarbonate, 0.5 µmol/l phosphate, 10 µmol/l iron, 7.0 µmol/l manganese, 5.0 µmol/l zinc, 25 µmol/l borium, 1.3 µmol/l copper, and 0.8 µmol/l molybdeen, ii) pH of 5.5-5.7, and iii) EC of 1.0 mS/cm.

Environmental stress factors which adversely affect flowering of *Gerbera* plants generally, such as changes in temperature, water stress and light conditions, have not been tested to determine if they may affect the degree of multi-petalledness per inflorescence or plant of the selected *multi-petalling-type Gerbera jamesonii* cultivars. The degree of multi-petalledness per *Gerbera jamesonii* inflorescence or plant may slightly decrease with increased temperatures, such as with a daily temperature average of 28°C rather than the preferred, daily temperature average of 24°C. Among *Gerbera jamesonii* cultivars in which the degree of multi-petalledness per inflorescence or plant may be adversely affected by temperature and/or light conditions, the degree of multi-petalledness can be restored with removal of the environmental stress factor(s).

The following examples are set forth as representative of the specific and preferred embodiments of the present invention. These examples are not to be construed as limiting the scope of the invention in any manner. It should be understood that many variations and modifications can be made while remaining within the spirit and scope of the invention.

### 5. Examples

### Example 1: Genetic Data

The data from about 30 to 50 crosses with *Gerbera jamesonii* indicate that the multi-petalled-type and globular shaped inflorescence trait is polygenic. Regardless of what the genetic basis for control of the multi-petalled-type and globular-shaped inflorescence trait in *Gerbera jamesonii* may be, these data demonstrate that the multi-petalled-type trait can be reproducibly and predictably introgressed into diverse *Gerbera jamesonii* genetic backgrounds.

### Example 2: Gerbera jamesonii cultivar 'Tersunrutas'

The present example of the invention comprises a new and distinct cultivar of *Gerbera jamesonii,* referred to by the cultivar name 'Tersunrutas'.

'Tersunrutas' originated from a hybridization program in Kudelstaart, The Netherlands, in 2002. The female parent was an unpatented *Gerbera jamesonii* designated 'MI00.009', and is characterized by its double-type and incurving, funnel-shaped flowering and mixed pink petals with green center petals. The male parent was an unpatented *Gerbera jamesonii* designated 'M98.502', and is characterized by it semi-double-type and incurving, funnel-shaped flowering, high petal count, and bi-color yellow petals, with an orange inner ring of petals and a dark center. The new cultivar 'Tersunrutas' was selected by the inventors as a single flowering plant within the progeny of the stated cross in a controlled environment in Kudelstaart, The Netherlands, on or about November of 2002.

The first asexual reproduction of 'Tersunrutas' was accomplished when vegetative cuttings were taken in January of 2003 in Kudelstaart, The Netherlands. The new cultivar 'Tersunrutas' is presently being propagated by cuttings and tissue culture. Horticultural examination of selected units initiated in 2003-2004 has demonstrated that the combination of characteristics as herein disclosed for 'Tersunrutas' are firmly fixed and are retained through successive generations of asexual reproduction. The new cultivar reproduces true-to-type.

The following traits have been repeatedly observed and are determined to be unique characteristics of the new cultivar 'Tersunrutas', which in combination distinguish this *Gerbera jamesonii* as a new and distinct cultivar:
1. inflorescences having i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence;
2. inflorescences having a globular shape;
3. inflorescences having a diameter of about 7.5 cm to 8.0 cm; and
4. inflorescences having a general pink tonality color (from a distance of 3 meters).

The new *Gerbera jamesonii* cultivar 'Tersunrutas' has not been observed under all possible environmental conditions. The phenotype of 'Tersunrutas' may vary with variations in environment such as temperature, light intensity, and day length without any change in the genotype of the plant. The following observations, measurements and comparisons describe plants of 'Tersunrutas' grown in Kudelstaart, The Netherlands, under greenhouse conditions which closely approximate those generally used in commercial practice.

Plants of 'Tersunrutas' differ from plants of the unpatented parents, 'MI00.009' and 'M98.502', in the following characteristics of Table 7:

**Table 7**

| **Trait** | **New Cultivar 'Tersunrutas'** | **Female Parent 'MI00.009' (unpatented)** | **Male Parent 'M98.502' (unpatented)** |
|---|---|---|---|
| Flowering Type | Multi-petalled-type | Double-type | Semi-double-type |
| Flower Head Shape | Globular | Incurving, funnel | Incurving, funnel |
| Flower Head Size | Small | Medium | Small |
| Flower Head Color | Pink | Pink mix | Bi-color yellow, with orange inner ring |
| Full of Partial Outer Petal Number per Inflorescence | At least 320, to about 550 | At least 250, to about 270 | At least 130, to about 170 |

Of the many *Gerbera jamesonii* commercial cultivars known to the inventors, there is no cultivar similar in comparison to 'Tersunrutas'.

In the following description, color references are made to the Royal Horticultural Society Color Chart (RHS), except where general colors of ordinary significance are used. The color values were determined at 9:30 a.m. on September 15, 2005, under artificial light in a greenhouse, in Kudelstaart, The Netherlands. The age of the plant described is about 4 months old.

### I. INFLORESCENCE:

A. Flower Head:

| Type: | Capitulum |
|---|---|
| Flowering Type: | Multi-petalled. |
| Diameter: | Small (about 7.5 to 8.0 cm). |
| Color (general tonality from a distance of 3 meters:) | Pink. |
| Shape: | Globular shaped due to combination of strong incurviness of outer florets, strong reflexing of the inner florets and the floret flag. |

| Involucre: | |
|---|---|
| Height from point of attachment of involucre to top of flower head: | Medium (about 25 mm) |
| Height: | Medium (about 8-10 mm). |
| Diameter: | Medium (about 40-45 mm). |
| Number of bracts: | Medium (about 86). |
| Longitudinal axis of inner rows: | Reflexing. |
| Anthocyanin: | Absent or very weak at tips. |
| Pubescence: | Medium. |

| Ray florets: | |
|---|---|
| Number: | Very high (472). |
| Overall Shape: | Obovate. |
| Longitudinal axis outer row: | Strongly incurving. |
| Longitudinal axis inner row: | Incurving. |
| Longitudinal axis of ray female floret: | Incurving. |
| Longitudinal axis of ray male floret: | Reflexing. |

| Outer ray floret: | |
|---|---|
| Cross section: | Convex. |
| Length: | Short-medium (about 35 mm). |
| Width: | Medium (about 10-12 mm). |
| Longitudinal folding: | Medium. |
| Angle of apex: | Obtuse. |
| Shape of apex: | Rounded |
| Incisions of apex: | 0-1. |
| Depth of incision: | Absent to very shallow. |
| Color (top side): | Red, RHS 37A. |
| Color (bottom side) | Red, RHS 37A. |
| Color distribution on inner side: | Uniform. |
| Edge of different color: | Present (0.5 mm, yellow, RHS 11B). |
| Striation: | Absent. |
| Claw spot: | Present (10%, yellow, RHS 4D). |

B. Disc florets:

| | |
|---|---|
| Number: | 250-280 |
| Disc diameter: | Small (about 15-17 mm). |
| Color (mature, upperside): | Pink (closest to red, RHS 41B) |
| Color (immature, top): | Green, RHS 138B to RHS 138C. |
| Main color upperside corolla: | Female flowers: pink (closest to red, RHS 41B). |
| | Male flowers: pink (closest to red, RHS 41B). |

C. Reproductive Organs:

| | |
|---|---|
| Style: | Main color distal part: yellow-white (RHS 158D). |
| Stigma: | Main color: light green-yellow (RHS 1D). |
| Anthers: | Main color: yellow (RHS 12A). |
| | Color of top relative to other parts is lighter. |
| | Longitudinal stripes are absent. |
| | Intensity of anthocyanin coloration is absent. |
| Pappus: | Main color: red-purple (RHS 72A). |
| | Color of top relative to other parts is identical. |
| | Level of top relative to closed disc florets: far below. |
| Fertility: | Fertility (male and female) as well as the seed setting is very poor. |

D. Peduncle:

| | |
|---|---|
| Length: | Long (about 58±3 cm). |
| Cross section: | Elliptic. |
| Tendency to fasciation: | Absent. |
| Thickness: | Thick. |
| Strength: | Strong. |
| Pubescence: | Dense. |
| Color: | Light yellow-green (RHS 144A). |
| Anthocyanin coloration: | At base: medium (gray-purple, RHS 183D). |
| | At top: absent. |
| Involucral bracts: | Absent. |

### II. PLANT:

A. General appearance:

| | |
|---|---|
| Height: | 35-40 cm (excluding any inflorescences). |
| Spread: | 60-70 cm |

B. Foliage:

| Leafblade: | |
|---|---|
| Length: | Medium (about 41±2 cm). |
| Width: | Medium (about 17±2 cm). |
| Thickness: | Medium. |
| Blistering: | Medium. |
| Pubescence: | On upper side (midrib excluded): medium. |
| Depth of cuts or incisions in | Basal part: deep. |
| leaf: | Central part: medium. |
| | Distal part: shallow. |
| Color: | Upper side: Medium green (RHS 141A) |
| | Bottom side: Green (RHS 138A). |
| Glossiness on upper side: | Weak. |
| Angle of apex: | Acute. |
| Shape of apex: | Pointed. |
| Margin of lobes: | Crenate. |
| Extensions of margin: | Large. |

| Petiole: | |
|---|---|
| Length: | Short (about 10±2 cm). |
| Color: | Yellow-green, RHS 144A. |
| Anthocyanin coloration: | Strong (greyed-red, RHS 178B). |

C. Disease/pest resistance/susceptibility:

| |
|---|
| No special disease/pest resistance/susceptibility. |

### Example 3: Gerbera jamesonii cultivar 'Tersram'

The present example of the invention comprises a new and distinct cultivar of *Gerbera jamesonii,* referred to by the cultivar name 'Tersram'.

'Tersram' originated from a hybridization program in Kudelstaart, The Netherlands, in 2003. The female parent was an unpatented *Gerbera jamesonii* designated 'CH01,602', and is characterized by its double-multi-type and incurving, funnel-shaped flowering and red petal color. The male parent was an uunpatented *Gerbera jamesonii* designated 'M02.502', and is characterized its by double-type and incurving, funnel-shaped flowering and red petal color. The new cultivar 'Tersram' was selected by the inventors as a single flowering plant within the progeny of the stated cross in a controlled environment in Kudelstaart, The Netherlands, on or about November of 2003.

The first asexual reproduction of 'Tersram' was accomplished when vegetative cuttings were taken in January of 2004 in Kudelstaart, The Netherlands. The new cultivar 'Tersram' is presently being propagated by cuttings and tissue culture. Horticultural examination of selected units initiated in 2004-2005 has demonstrated that the combination of characteristics as herein disclosed for 'Tersram' are firmly fixed and are retained through successive generations of asexual reproduction. The new cultivar reproduces true-to-type.

The following traits have been repeatedly observed and are determined to be unique characteristics of the new cultivar 'Tersram', which in combination distinguish this *Gerbera jamesonii* as a new and distinct cultivar:
1. inflorescences having i) at least 320, to about 550, full or partial outer petals (ray florets) per inflorescence, ii) at least .150, to about 300, full or partial inner petals (disc florets) per inflorescence, and iii) at least 520, to about 800, full or partial total petals (total florets) per inflorescence;
2. inflorescences having a globular shape;
3. inflorescences having a diameter of about 7.5 cm to 8.0 cm; and
4. inflorescences having a general red tonality color (from a distance of 3 meters).

The new *Gerbera jamesonii* cultivar 'Tersram' has not been observed under all possible environmental conditions. The phenotype of 'Tersram' may vary with variations in environment such as temperature, light intensity, and day length without any change in the genotype of the plant. The following observations, measurements and comparisons describe plants of 'Tersram' grown in Kudelstaart, The Netherlands, under greenhouse conditions which closely approximate those generally used in commercial practice.

Plants of 'Tersram' differ from plants of the unpatented parents, 'CH01.602' and 'M02.502', in the following characteristics of Table 8:

**Table 8**

| **Trait** | **New Cultivar 'Tersram'** | **Female Parent 'CH01.602' (unpatented)** | **Male Parent 'M02.502' (unpatented)** |
|---|---|---|---|
| Flowering Type | Multi-petalled-type | Double-multi-type | Double-type |
| Flower Head Shape | Globular | Incurving, funnel | Incurving, funnel |
| Flower Head Size | Small | Medium | Medium |
| Full of Partial Outer Petal Number per Inflorescence | At least 320, to about 550 | At least 270, to about 320 | At least 250, to about 270 |

Of the many *Gerbera jamesonii* commercial cultivars known to the inventors, there is no cultivar similar in comparison to 'Tersram'.

In the following description, color references are made to the Royal Horticultural Society Color Chart (RHS), except where general colors of ordinary significance are used. The color values were determined at 10:30 a.m. on September 15, 2005, under artificial light in a greenhouse, in Kudelstaart, The Netherlands. The age of the plant described is about 4 months old.

### I. INFLORESCENCE:

A. Flower Head:

| | |
|---|---|
| Type: | Capitulum |
| Flowering Type: | Multi-petalled. |
| Diameter: | Small (about 7.5 to 8.0 cm). |
| Color (general tonality from a distance of 3 meters:) | Pink. |
| Shape: | Globular shaped due to combination of strong incurviness of outer florets, strong reflexing of the inner florets and the floret flag. |

| Involucre: | |
|---|---|
| Height from point of attachment of involucre to top of flower head: | Medium (about 25-30 mm). |
| Height: | Medium (about 8-10 mm). |
| Diameter: | Medium (about 40-45 mm). |
| Number of bracts: | Medium (about 81). |
| Longitudinal axis of inner rows: | Reflexing. |
| Anthocyanin: | Present. |
| Pubescence: | Medium. |

| Ray florets: | |
|---|---|
| Number: | Very high (419). |
| Overall Shape: | Obovate. |
| Longitudinal axis outer row: | Strongly incurving. |
| Longitudinal axis inner row: | Incurving. |
| Longitudinal axis of ray female floret: | Incurving. |
| Longitudinal axis of ray male floret: | Reflexing. |

| Outer ray floret: | |
|---|---|
| Cross section: | Convex. |
| Length: | Short-medium (about 30-35 mm). |
| Width: | Width: broad (about 12-14 mm). |
| Longitudinal folding: | Strong. |
| Angle of apex: | Broadly obtuse. |
| Shape of apex: | Rounded |
| Incisions of apex: | 1-2. |
| Depth of incision: | Shallow. |
| Color (top side): | Red, RHS 44A. |
| Color (bottom side) | Red, RHS 44A. |
| Color distribution on inner side: | Uniform. |
| Edge of different color: | Absent. |
| Striation: | Absent. |
| Claw spot: | Present (5%, yellow, RHS 8A). |

B. Disc florets:

| | |
|---|---|
| Number: | 190-220 |
| Disc diameter: | Small (about 15 mm). |
| Color (mature, upperside): | Red (RHS 44A). |
| Color (immature, top): | Red (RHS 47A). |
| Main color upperside corolla: | Female flowers: red (RHS 44A). |
| | Male flowers: red (RHS 44A). |

C. Reproductive Organs:

| | |
|---|---|
| Style: | Main color distal part: light yellow (RHS 4C). |
| Stigma: | Main color: light yellow (RHS 4C). |
| Anthers: | Main color: bottom (50%) brown (closest to RHS 166C), 30% greyed-purple (RHS 187A), top yellow/gray (RHS 160A). |
| | Color of top relative to other parts is lighter. |
| | Longitudinal stripes are absent. |
| | Intensity of anthocyanin coloration is strong. |
| Pappus: | Main color: red-purple (RHS 71A). |
| | Color of top relative to other parts is identical. |
| | Level of top relative to closed disc florets: far below. |
| Fertility: | Fertility (male and female) as well as the seed setting is very poor. |

D. Peduncle:

| | |
|---|---|
| Length: | Long (about 62±3 cm). |
| Cross section: | Elliptic. |
| Tendency to fasciation: | Present. |
| Thickness: | Medium. |
| Strength: | Medium. |
| Pubescence: | Medium. |
| Color: | Light green (RHS 144A). |
| Anthocyanin coloration: | At base: strong, gray-purple (RHS 183B). |
| | At top: absent. |
| Involucral bracts: | Absent. |

### II. PLANT:

A. General appearance:

| | |
|---|---|
| Height: | 35-40 cm (excluding any inflorescences). |
| Spread: | 65-75 cm |

B. Foliage:

| Leaf blade: | |
|---|---|
| Length: | Medium (about 35±2 cm). |
| Width: | Medium (about 16±1 cm). |
| Thickness: | Medium. |
| Blistering: | Medium. |
| Pubescence: | On upper side (midrib excluded): medium. |
| Depth of cuts or incisions in leaf: | Basal part: deep. |
| | Central part: medium. |
| | Distal part: medium. |
| Color: | Upper side: medium green (RHS 141A). |
| | Bottom side: green (RHS 138A). |
| Glossiness on upper side: | Absent. |
| Angle of apex: | Acute. |
| Shape of apex: | Rounded. |
| Margin of lobes: | Serrate. |
| Extensions of margin: | Medium. |

| Petiole: | |
|---|---|
| Length: | Medium (about 13±2 cm). |
| Color: | Yellow-green, RHS 144A. |
| Anthocyanin coloration: | Strong (gray-purple, RHS 183A). |

C. Disease/pest resistance/susceptibility:

| |
|---|
| No special disease/pest resistance/susceptibility. |

### Example 4: Gerbera jamesonii cultivar 'Tersunev'

The present example of the invention comprises a new and distinct cultivar of *Gerbera jamesonii,* referred to by the cultivar name 'Tersunev'.

'Tersunev' originated from a hybridization program in Kudelstaart, The Netherlands, in 2003. The female parent was an unpatented *Gerbera jamesonii* cultivar designated 'VA3298', and is characterized by its semi-double-type and incurving, flat-shaped flowering and cream petal color with green center petal color. The male parent was an unpatented *Gerbera jamesonii* cultivar designated 'CH02.651', and is characterized by its double-multi-type and rounded-shaped flowering and red petal color. The new cultivar Tersunev' was selected by the inventors as a single flowering plant within the progeny of the stated cross in a controlled environment in Kudelstaart, The Netherlands, on or about October of 2003.

The first asexual reproduction of 'Tersunev' was accomplished when vegetative cuttings were taken in January of 2004 in Kudelstaart, The Netherlands. The new cultivar 'Tersunev' is presently being propagated by cuttings and tissue culture. Horticultural examination of selected units initiated in 2004-2005 has demonstrated that the combination of characteristics as herein disclosed for 'Tersunev' are firmly fixed and are retained through successive generations of asexual reproduction. The new cultivar reproduces true-to-type.

The following traits have been repeatedly observed and are determined to be unique characteristics of the new cultivar 'Tersunev', which in combination distinguish this *Gerbera* as a new and distinct cultivar:
1. inflorescences having i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence;
2. inflorescences having a globular shape;
3. inflorescences having a diameter of about 6.5 cm to 7.0 cm; and
4. inflorescences having a general purple tonality color (from a distance of 3 meters).

The new *Gerbera jamesonii* cultivar 'Tersunev' has not been observed under all possible environmental conditions. The phenotype of 'Tersunev' may vary with variations in environment such as temperature, light intensity, and day length without any change in the genotype of the plant. The following observations, measurements and comparisons describe plants of 'Tersunev' grown in Kudelstaart, The Netherlands, under greenhouse conditions which closely approximate those generally used in commercial practice.

Plants of 'Tersunev' differ from plants of the unpatented parents, 'VA3298' and 'CH02.651', in the following characteristics of Table 9:

**Table 9**

| **Trait** | **New Cultivar 'Tersunev'** | **Female Parent 'VA3298' (unpatented)** | **Male Parent 'CH02.651' (unpatented** |
|---|---|---|---|
| Flowering Type | Multi-petalled-type | Semi-double-type | Double-multi-type |
| Flower Head Shape | Globular | Incurving, flat | Rounded, but not globular |
| Flower Head Size | Small | Medium | Medium |
| Flower Head General Color | Purple | Cream | Red |
| Full of Partial Outer Petal Number per Inflorescence | At least 320, to about 550 | At least 130, to about 170 | At least 270, to about 320 |

Of the many *Gerbera jamesonii* commercial cultivars known to the inventors, there is no cultivar similar in comparison to 'Tersunev'.

In the following description, color references are made to the Royal Horticultural Society Color Chart (RHS), except where general colors of ordinary significance are used. The color values were determined at 11:30 a.m. on September 15, 2005, under artificial light in a greenhouse, in Kudelstaart, The Netherlands. The age of the plant described is about 4 months old.

### I. INFLORESCENCE:

A. Flower Head:

| | |
|---|---|
| Type: | Capitulum |
| Flowering Type: | Multi-petalled. |
| Diameter: | Small (about 6.5 to 7.0 cm). |
| Color (general tonality from a distance of 3 meters:) | Purple. |
| Shape: | Globular shaped due to combination of strong incurviness of outer florets, strong reflexing of the inner florets and the floret flag. |

| Involucre: | |
|---|---|
| Height from point of attachment of involucre to top of flower | Low (about 20-25 mm). |
| head: | |
| Height: | Low (about 6-8 mm). |
| Diameter: | Small (about 35-40 mm). |
| Number of bracts: | Medium (about 80). |
| Longitudinal axis of inner rows: | Reflexing. |
| Anthocyanin: | Present. |
| Pubescence: | Medium. |

| Ray florets: | |
|---|---|
| Number: | Very high (335). |
| Overall Shape: | Obovate. |
| Longitudinal axis outer row: | Strongly incurving. |
| Longitudinal axis inner row: | Incurving. |
| Longitudinal axis of ray female floret: | Incurving. |
| Longitudinal axis of ray male floret: | Reflexing. |

| Outer ray floret: | |
|---|---|
| Cross section: | Convex. |
| Length: | Short-medium (about 30-33 mm). |
| Width: | Medium (about 9-10 mm). |
| Longitudinal folding: | Strong. |
| Angle of apex: | Obtuse. |
| Shape of apex: | Rounded. |
| Incisions of apex: | 0-2. |
| Depth of incision: | Shallow. |
| Color (top side): | Red-purple, RHS 57B. |
| Color (bottom side) | Red-purple, RHS 57B. |
| Color distribution on inner side: | Uniform. |
| Edge of different color: | Absent. |
| Striation: | Absent. |
| Claw spot: | Present (5%, red-purple, RHS 65D). |

B. Disc florets:

| | |
|---|---|
| Number: | 185-210 |
| Disc diameter: | Small (about 19 mm). |
| Color (mature, upperside): | Purple (closest to RHS 57B). |
| Color (immature, top): | Red-purple, RHS 60B. |
| Main color upperside corolla: | Female flowers: purple (closest to RHS 57B). |
| | Male flowers: purple (closest to RHS 57B). |

C. Reproductive Organs:

| | |
|---|---|
| Style: | Main color distal part: yellow-white (RHS 158D). |
| Stigma: | Main color: yellow-white (RHS 158D). |
| Anthers: | Main color: brown (closest to RHS 177A). |
| | Color of top relative to other parts is lighter. |
| | Longitudinal stripes are absent. |
| | Intensity of anthocyanin coloration is strong. |
| Pappus: | Main color: light yellow (RHS 11C). |
| | Color of top relative to other parts is darker (grey yellow RHS 161B). |
| | Level of top relative to closed disc florets: far below. |
| Fertility: | Fertility (male and female) as well as the seed setting is very poor. |

D. Peduncle:

| | |
|---|---|
| Length: | Long (about 58±3 cm). |
| Cross section: | Elliptic. |
| Tendency to fasciation: | Present. |
| Thickness: | Thick. |
| Strength: | Strong. |
| Pubescence: | Medium. |
| Color: | Light green (RHS 144A). |
| Anthocyanin coloration: | At base: absent. |
| | At top: absent. |
| Involucral bracts: | Absent. |

### II. PLANT:

A. General appearance:

| | |
|---|---|
| Height: | 35-40 cm (excluding any inflorescences). |
| Spread: | 60-70 cm |

B. Foliage:

| Leafblade: | |
|---|---|
| Length: | Medium (about 34±2 cm). |
| Width: | Medium (about 15±2 cm). |
| Thickness: | Medium. |
| Blistering: | Medium. |
| Pubescence: | On upper side (midrib excluded): sparse. |
| Depth of cuts or incisions in leaf: | Basal part: shallow. |
| | Central part: medium. |
| | Distal part: shallow. |
| Color: | Upper side: medium green (RHS 141A) |
| | Bottom side: green (RHS 138A). |
| Glossiness on upper side: | Weak. |
| Angle of apex: | Acute. |
| Shape of apex: | Pointed. |
| Margin of lobes: | Crenate. |
| Extensions of margin: | Large. |

| Petiole: | |
|---|---|
| Length: | Medium (about 13±1 cm). |
| Color: | RHS 144A. |
| Anthocyanin coloration: | Absent. |

C. Disease/pest resistance/susceptibility:

| |
|---|
| No special disease/pest resistance/susceptibility. |

### Example 5: Gerbera jamesonii cultivar 'Ternoom'

The present example of the invention comprises a new and distinct cultivar of *Gerbera jamesonii,* referred to by the cultivar name 'Ternoom'.

'Ternoom' originated from a hybridization program in Kudelstaart, The Netherlands, in 2003. The female parent was an unpatented *Gerbera jamesonii* designated 'CH02.645', and is characterized by its double-multi-type and rounded-shaped flowering and orange petal color. The male parent was an unpatented *Gerbera jamesonni* designated 'CH02.653', and is characterized by its double-multi-type and rounded-shaped flowering and pink petal color with a green center. The new cultivar 'Ternoom' was selected by the inventors from the progeny of the stated cross in a controlled environment in Kudelstaart, The Netherlands, on or about November of 2003.

The first asexual reproduction of 'Ternoom' was accomplished when vegetative cuttings were taken in January of 2004 in Kudelstaart, The Netherlands. The new cultivar 'Ternoom' is presently being propagated by cuttings and tissue culture. Horticultural examination of selected units initiated in 2004-2005 has demonstrated that the combination of characteristics as herein disclosed for 'Ternoom' are firmly fixed and are retained through successive generations of asexual reproduction. The new cultivar reproduces true-to-type.

The following traits have been repeatedly observed and are determined to be unique characteristics of the new cultivar 'Temoom', which in combination distinguish this *Gerbera jamesonii* as a new and distinct cultivar:
1. inflorescences having i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence;
2. inflorescences having a globular shape;
3. inflorescences having a diameter of about 7.0 cm to 7.5 cm; and
4. inflorescences having a general light yellow-orange tonality color (from a distance of 3 meters).

The new *Gerbera jamesonii* cultivar 'Ternoom' has not been observed under all possible environmental conditions. The phenotype of 'Ternoom' may vary with variations in environment such as temperature, light intensity, and day length without any change in the genotype of the plant. The following observations, measurements and comparisons describe plants of 'Ternoom' grown in Kudelstaart, The Netherlands, under greenhouse conditions which closely approximate those generally used in commercial practice.

Plants of 'Ternoom' differ from plants of the unpatented parents, 'CH02.645' and 'CH02.653', in the following characteristics of Table 10:

**Table 10**

| **Trait** | **New Cultivar 'Ternoom'** | **Female Parent 'CH02.645' (unpatented)** | **Male Parent 'CH02.653' (unpatented)** |
|---|---|---|---|
| Flowering Type | Multi-petalled-type | Double-multi-type | Double-multi-type |
| Flower Head Shape | Globular | Rounded, but not globular | Rounded, but not globular |
| Flower Head Size | Small | Medium | Medium |
| Flower Head General Color | Light yellow-orange | Orange | Pink with green center |
| Full of Partial Outer Petal Number per Inflorescence | At least 320, to about 550 | At least 270, to about 320 | At least 270, to about 320 |

Of the many *Gerbera jamesonii* commercial cultivars known to the inventors, there is no cultivar similar in comparison to 'Ternoom'.

In the following description, color references are made to the Royal Horticultural Society Color Chart (RHS), except where general colors of ordinary significance are used. The color values were determined at 12:30 p.m. on September 15, 2005, under artificial light in a greenhouse in Kudelstaart, The Netherlands. The age of the plant described is about 4 months old.

### I. INFLORESCENCE:

A. Flower Head:

| | |
|---|---|
| Type: | Capitulum |
| Flowering Type: | Multi-petalled. |
| Diameter: | Small (about 7.0 to 7.5 cm). |
| Color (general tonality from a distance of 3 meters:) | Light yellow-orange. |
| Shape: | Globular shaped due to combination of strong incurviness of outer florets, strong reflexing of the inner florets and the floret flag. |

| Involucre: | |
|---|---|
| Height from point of attachment of involucre to top of flower head: | Medium (about 25 mm). |
| Height: | Medium (about 8 mm). |
| Diameter: | Medium (about 40-45 mm). |
| Number of bracts: | High (about 100). |
| Longitudinal axis of inner rows: | Reflexing. |
| Anthocyanin: | Absent or very weak at tips. |
| Pubescence: | Medium. |

| Ray florets: | |
|---|---|
| Number: | Very high (492). |
| Overall Shape: | Obovate. |
| Longitudinal axis outer row: | Strongly incurving. |
| Longitudinal axis inner row: | Incurving. |
| Longitudinal axis of ray female floret: | Incurving. |
| Longitudinal axis of ray male floret: | Reflexing. |

| Outer ray floret: | |
|---|---|
| Cross section: | Convex. |
| Length: | Short (about 25-30 mm). |
| Width: | Medium (about 9-10 mm). |
| Longitudinal folding: | Medium. |
| Angle of apex: | Obtuse. |
| Shape of apex: | Rounded. |
| Incisions of apex: | 0-1. |
| Depth of incision: | Shallow. |
| Color (top side): | RHS 19B. |
| Color (bottom side) | RHS 19B. |
| Color distribution on inner side: | Uniform. |
| Edge of different color: | Absent. |
| Striation: | Absent. |
| Claw spot: | Absent. |

B. Disc florets:

| | |
|---|---|
| Number: | 195-220 |
| Disc diameter: | Small (about 18-19 mm). |
| Color (mature, upperside): | Orange (RHS 29B). |
| Color (immature, top): | Green, RHS 142A. |
| Main color upperside corolla: | Female flowers: yellow-orange (RHS 22C). |
| | Male flowers: Orange (RHS 29B). |

C. Reproductive Organs:

| | |
|---|---|
| Style: | Main color distal part: light yellow (RHS 4D). |
| Stigma: | Main color: light yellow (RHS 4C). |
| Anthers: | Main color: yellow-orange (RHS 17C). |
| | Color of top relative to other parts is darker. |
| | Longitudinal stripes are present. |
| | Intensity of anthocyanin coloration is weak. |
| Pappus: | Main color: light yellow (RHS 2B). |
| | Color of top relative to other parts is darker (red-purple, RHS 72A). |
| | Level of top relative to closed disc florets: far below. |
| Fertility: | Fertility (male and female) as well as the seed setting is very poor. |

D. Peduncle:

| | |
|---|---|
| Length: | Long (about 56±3 cm). |
| Cross section: | Round. |
| Tendency to fasciation: | Absent. |
| Thickness: | Medium. |
| Strength: | Medium. |
| Pubescence: | Dense. |
| Color: | Light green (RHS 144A). |
| Anthocyanin coloration: | At base: weak (greyed-green, RHS 197A). |
| | At top: absent. |
| Involucral bracts: | Absent. |

### II. PLANT:

A. General appearance:

| | |
|---|---|
| Height: | 30-35 cm (excluding any inflorescences). |
| Spread: | 50-60 cm |

B. Foliage:

| Leaf blade: | |
|---|---|
| Length: | Short (about 21±2 cm). |
| Width: | Narrow (about 7.5±1 cm). |
| Thickness: | Thick. |
| Blistering: | Strong. |
| Pubescence: | On upper side (midrib excluded): medium. |
| Depth of cuts or incisions in leaf: | Basal part: deep. |
| | Central part: deep. |
| | Distal part: medium. |
| Color: | Upper side: Dark green (RHS 139A) |
| | Bottom side: RHS 138A. |
| Glossiness on upper side: | Strong. |
| Angle of apex: | Acute. |
| Shape of apex: | Rounded. |
| Margin of lobes: | Serrate. |
| Extensions of margin: | Small. |

| Petiole: | |
|---|---|
| Length: | Long (about 17±1 cm). |
| Color: | Yellow-green, RHS 146B. |
| Anthocyanin coloration: | Weak (gray-orange, RHS 174A). |

C. Disease/pest resistance/susceptibility:

| |
|---|
| No special disease/pest resistance/susceptibility. |

### Example 6: Gerbera jamesonii cultivar 'Terlob'

The present example of the invention comprises a new and distinct cultivar of *Gerbera* jamesonii, referred to by the cultivar name 'Terlob'.

'Terlob' originated from a hybridization program in Kudelstaart, The Netherlands, in 2004. The female parent was an unpatented *Gerbera jamesonii* designated 'CH04.635' (a seedling taken from the new 'Tersunev' seedling selection), and is characterized by its multi-petalled-type and globular-shaped flowering and purple petal color. The male parent was an unpatented *Gerbera jamesonii* designated 'M93.002', and is characterized by its double-multi-type and rounded-shaped flowering and mixed pink petal color. The new cultivar was selected by the inventors as a single flowering plant within the cross of the stated cross in a controlled environment in Kudelstaart, The Netherlands, on or about October of 2004.

The first asexual reproduction of'Terlob' was accomplished when vegetative cuttings were taken in December of 2004 in Kudelstaart, The Netherlands. The new cultivar 'Terlob' is presently being propagated by cuttings and tissue culture. Horticultural examination of selected units initiated in 2004-2005 has demonstrated that the combination of characteristics as herein disclosed for 'Terlob' are firmly fixed and are retained through successive generations of asexual reproduction. The new cultivar reproduces true-to-type. Further, the continued crossing showed the increased effect of the number of petals and the specific formation of the petals.

The following traits have been repeatedly observed and are determined to be unique characteristics of the new cultivar 'Terlob', which in combination distinguish this *Gerbera jamesonii* as a new and distinct cultivar:
1. inflorescences having i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence;
2. inflorescences having a globular shape;
3. inflorescences having a diameter of about 6.0 cm to 6.5 cm; and
4. inflorescences having a general purple tonality color (from a distance of 3 meters).

The new *Gerbera jamesonii* cultivar 'Terlob' has not been observed under all possible environmental conditions. The phenotype of 'Terlob' may vary with variations in environment such as temperature, light intensity, and day length without any change in the genotype of the plant. The following observations, measurements and comparisons describe plants of 'Terlob' grown in Kudelstaart, The Netherlands, under greenhouse conditions which closely approximate those generally used in commercial practice.

Plants of 'Terlob' differ from plants of the unpatented parents, 'CH04.625' and 'M93.002', in the following characteristics of Table 11:

**Table 11**

| **Trait** | **New Cultivar 'Terlob'** | **Female Parent 'CH04.625' (unpatented)** | **Male Parent 'M93.002' (unpatented)** |
|---|---|---|---|
| Flowering Type | Multi-petalled-type | Multi-petalled-type | Double-multi-type |
| Flower Head Shape | Globular | Globular | Rounded, but not globular |
| Flower Head Size | Small | Small | Medium |
| Flower Head General Color | Purple | Purple | Pink Mix |
| Full of Partial Outer Petal Number per Inflorescence | At least 320, to about 550 | At least 320, to about 550 | At least 270, to about 320 |

Of the many *Gerbera jamesonii* commercial cultivars known to the inventors, there is no cultivar similar in comparison to 'Terlob'.

In the following description, color references are made to the Royal Horticultural Society Color Chart (RHS), except where colors of ordinary significance are used. The color values were determined at 1:30 p.m. on September 15, 2005, under artificial light in a greenhouse in Kudelstaart, The Netherlands. The age of the plant described is about 4 months.

### I. INFLORESCENCE:

A. Flower Head:

| | |
|---|---|
| Type: | Capitulum |
| Flowering Type: | Multi-petalled. |
| Diameter: | Small (about 6.0 to 6.5 cm). |
| Color (general tonality from a distance of 3 meters:) | Purple. |
| Shape: | Globular shaped due to combination of strong incurviness of outer florets, strong reflexing of the inner florets and the floret flag. |

| Involucre: | |
|---|---|
| Height from point of attachment of involucre to top of flower head: | Low (about 20 mm). |
| Height: | Low (about 3-4 mm). |
| Diameter: | Small (about 35-40 mm). |
| Number of bracts: | Medium (about 73). |
| Longitudinal axis of inner rows: | Reflexing. |
| Anthocyanin: | Present. |
| Pubescence: | Medium. |

| Ray florets: | |
|---|---|
| Number: | Very high (492). |
| Overall Shape: | Obovate. |
| Longitudinal axis outer row: | Strongly incurving. |
| Longitudinal axis inner row: | Incurving. |
| Longitudinal axis of ray female floret: | Incurving. |
| Longitudinal axis of ray male floret: | Reflexing. |

| Outer ray floret: | |
|---|---|
| Cross section: | Concave. |
| Length: | Short (about 25-27 mm). |
| Width: | Medium (about 7-9 mm). |
| Longitudinal folding: | Strong. |
| Angle of apex: | Broadly obtuse. |
| Shape of apex: | Rounded. |
| Incisions of apex: | 1-2. |
| Depth of incision: | Medium-deep. |
| Color (top side): | Red-purple, RHS 63A. |
| Color (bottom side) | Red-purple, RHS 70C. |
| Color distribution on inner side: | Uniform. |
| Edge of different color: | Absent. |
| Striation: | Absent. |
| Claw spot: | (5%, white, RHS 155A). |

B. Disc florets:

| | |
|---|---|
| Number: | 165-195 |
| Disc diameter: | Small (about 15 mm). |
| Color (mature, upperside): | Purple (closest to RHS 60B). |
| Color (immature, top): | Red-purple, RHS 70B. |
| Main color upperside corolla: | Female flowers: purple (closest to RHS 63A). |
| | Male flowers: purple (closest to RHS 60B). |

C. Reproductive Organs:

| | |
|---|---|
| Style: | Main color distal part: white (RHS 155C). |
| Stigma: | Main color: pink (closest to RHS 63C). |
| Anthers: | Main color: brown (gray-purple, RHS 187A). |
| | Color of top relative to other parts is lighter. |
| | Longitudinal stripes are present. |
| | Intensity of anthocyanin coloration is strong. |
| Pappus: | Main color: light yellow-green (RHS 154D). |
| | Color of top relative to other parts is darker (red-purple RHS 61A). |
| | Level of top relative to closed disc florets: far below. |
| Fertility: | Fertility (male and female) as well as the seed setting is very poor. |

D. Peduncle:

| | |
|---|---|
| Length: | Medium (about 48±5 cm). |
| Cross section: | Elliptic. |
| Tendency to fasciation: | Present. |
| Thickness: | Thick. |
| Strength: | Strong. |
| Pubescence: | Medium. |
| Color: | Light yellow-green (RHS 144A). |
| Anthocyanin coloration: | At base: weak (greyed-brown, RHS 199B). |
| | At top: absent. |
| Involucral bracts: | Absent. |

### II. PLANT:

A. General appearance:

| | |
|---|---|
| Height: | 35-40 cm (excluding any inflorescences). |
| Spread: | 60-70 cm |

B. Foliage:

| Leafblade: | |
|---|---|
| Length: | Medium (about 32±2 cm). |
| Width: | Medium (about 19±1 cm).. |
| Thickness: | Medium. |
| Blistering: | Medium. |
| Pubescence: | On upper side (midrib excluded): sparse. |
| Depth of cuts or incisions in leaf: | Basal part: deep. |
| | Central part: deep. |
| | Distal part: medium. |
| Color: | Upper side: medium green (RHS 141 A) |
| | Bottom side: green, RHS 138A. |
| Glossiness on upper side: | Weak. |
| Angle of apex: | Acute. |
| Shape of apex: | Rounded. |
| Margin of lobes: | Serrate. |
| Extensions of margin: | Medium |

| Petiole: | |
|---|---|
| Length: | Long (about 20±2 cm). |
| Color: | Yellow-green, RHS 144A. |
| Anthocyanin coloration: | Weak (gray-orange, RHS 177B). |

C. Disease/pest resistance/susceptibility:

| |
|---|
| No special disease/pest resistance/susceptibility. |

The examples described herein are illustrative of the present invention and are not intended to be limitations thereon. Different embodiments of the present invention have been described according to the present invention. Many modifications and variations may be made to the methods and plants described and illustrated herein without departing from the spirit and scope of the invention.

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention, which is defined by the following claims. All publications and patent applications mentioned in this specification are indicative of the level of skill of those in the art to which the invention pertains.

All publications and patent applications are herein incorporated by reference to the same extent as if each individual publication or patent application were specifically and individually indicated to be incorporated by reference in its entirety.

## Claims

1. A *Gerbera jamesonii* plant, with one or more multi-petalled-type and globular-shaped inflorescences at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence.

2. The *Gerbera* plant according to claim 1, wherein said *Gerbera* species is selected from the group consisiting of *G. jamesonii, G. viridifolia, G. serrata* and *G. ambigua.*

3. The *Gerbera jamesonii* plant according to claim 1, wherein said plant is selected from the group consisting of the *Gerbera jamesonii* cultivars 'Tersunrutas', 'Tersram', 'Tersunev', 'Ternoom' and 'Terlob'.

4. The *Gerbera jamesonii* plant according to claim 1, wherein substantially all the inflorescences produced by said plant are multi-petalled and globular-shaped.

5. A multi-petalled-type and globular shaped *Gerbera jamesonii* plant wherein a multi-petalled-type inflorescence has i) at least 320, 330, 340, 350, 360, 370, 380, 390, 400, 410, 420, 430, 440, 450, 460, 470, 480, 490, 500, 510, 520, 530, 540 550, any integer between 320 and 550 or more than 550 full or partial outer petals (ray florets) per inflorescence, ii) at least 150, 160, 170, 180, 190, 200, 210, 220, 230, 240, 250, 260, 270, 280, 290, 300, any integer between 150 and 300, or more than 300 full or partial inner petals (disc florets) per inflorescence, and iii) at least 520,530, 540, 550, 560, 570, 580, 590, 600, 610, 620, 630, 640, 650, 660, 670, 680, 690, 700, 710, 720, 730, 740, 750, 760, 770, 780, 790, 800, any integer between 520 and 800, or more than 800 full or partial total petals (total florets) per inflorescence.

6. A method of breeding a multi-petalled-type and globular-shaped *Gerbera jamesonii* plant that produces one or more inflorescences having i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, comprising the steps of:
a. crossing a first single-type plant having the multi-petalled and globular shape trait in its genetic background, either as the male or female parent to:
i. a semi-double-type plant;
ii. a double-type plant;
iii. a double-multi-type plant; or
iv. a second single-type plant having the multi-petalled and globular shape trait in its genetic background;
b. selecting F1 progeny that are single-type, semi-double-type, double-type or double-multi-type;
c. crossing said F1 progeny that are single-type, semi-double-type, double-type or double-multi-type to:
i. a second semi-double-type plant;
ii. a second double-type plant;
iii. a second double-multi-type plant;
iv. a third single-type plant having the multi-petalled and globular shape trait in its genetic background; and
d. selecting multi-petalled and globular shaped flowering progeny.

7. The method according to claim 6, wherein said first, second and third single-type plants, or first and second semi-double-type, double-type or double-multi-type plants are the same or different cultivars.

8. A method of breeding a multi-petalled-type and globular-shaped *Gerbera jamesonii* plant that produces one or more inflorescences at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, comprising the steps of:
a. crossing a first semi-double-type, either as the male or female parent to:
i. a second semi-double-type plant;
ii. a double-type plant;
iii. a double-multi-type plant;
iv. a single-type plant having the multi-petalled and globular shape trait in its genetic background; or
v. a single-type plant with no known multi-petalled and globular shape trait in its genetic background;
b. selecting F1 progeny that are single-type, semi-double-type, double-type or double-multi-type;
c. crossing said F1 progeny that are single-type, semi-double-type, double-type or double-multi-type to:
i. a second double-type plant;
ii. a second double-multi-type plant;
iii. a third semi-double-type plant;
iv. a second single-type plant having the multi-petalled and globular shape trait in its genetic background; or
v. a second single-type plant with no known multi-petalled and globular shape trait in its genetic background; and
d. selecting multi-petalled and globular shaped flowering progeny.

9. The method according to claim 8, wherein said first and second single-type, double-type or double-multi-type plants, or first, second and third semi-double-type plants are same or different cultivars.

10. A method of breeding a multi-petalled-type and globular shaped *Gerbera jamesonii* plant that produce one or more inflorescences with at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, comprising the steps of:
a. crossing a first double-type plant, either as the male or female parent to:
i. a semi-double-type plant;
ii. a double-multi-type plant;
ii. a second double-type plant;
iv. a single-type plant having the multi-petalled and globular shape trait in its genetic background; or
v. a single-type plant with no known multi-petalled and globular shape trait in its genetic background;
b. selecting F1 progeny that are single-type, semi-double-type, double-type or double-multi-type;
c. crossing said F1 progeny that are single-type, semi-double-type, double-type or double-multi-type to:
i. a third double-type plant;
ii. a second semi-double-type plant;
iii. a second double-multi-type plant;
iv. a second single-type plant having the multi-petalled and globular shaped trait in its genetic background; or
v. a second single-type plant with no known multi-petalled and globular shaped trait in its genetic background; and
d. selecting multi-petalled-type and globular shaped flowering progeny.

11. The method according to claim 10, wherein said first, second and third double-type plants, or first and second single-type, semi-double-type or double-multi-type plants are the same or different cultivars.

12. A method of breeding a multi-petalled-type and globular shaped *Gerbera jamesonii* plants that produce one or more inflorescences with i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, comprising the steps of:
a. crossing a first double-multi-type plant, either as the male or female parent to:
i. a semi-double-type plant;
ii. a double-type plant;
iii. a single-type plant having the multi-petalled and globular shape trait in its genetic background;
iv. a single-type plant with no known multi-petalled and globular shape trait in its genetic background; or
v. a second double-multi-type plant;
b. selecting F1 progeny that are single-type, semi-double-type, double-type or double-multi-type;
c. crossing said F1 progeny that are single-type, semi-double-type, double-type or double-multi-type to:
i. a second semi-double-type plant;
ii. a second double-type plant;
iii. a second single-type plant having the multi-petalled and globular shaped trait in its genetic background;
iv. a second single-type plant with no known multi-petalled and globular shaped trait in its genetic background; or
v. a third double-multi-type plant; and
d. selecting multi-petalled-type and globular shaped flowering progeny.

13. The method according to claim 12, wherein said first, second and third double-multi-type plants, or first and second single-type, semi-double-type or double-type plants are the same or different cultivars.

14. A method of breeding a multi-petalled-type and globular shaped *Gerbera jamesonii* plants that produce one or more inflorescences with i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, comprising the steps of:
a. crossing a first multi-petalled-type and globular shaped plant, either as the male or female parent to:
i. a semi-double-type plant;
ii. a double-type plant;
iii. a double-multi-type plant;
iv. a single-type plant having the multi-petalled and globular shape trait in its genetic background;
v. a single-type plant with no known multi-petalled and globular shape trait in its genetic background; or
vi. a second multi-petalled-type and globular shaped plant;
b. selecting from F1:
i. multi-petalled and globular-shaped flowering progeny; or
ii. semi-double-type, double-type or double-multi-type progeny;
c. crossing said F1 progeny that are semi-double-type, double-type or double-multi-type to:
i. a second semi-double-type plant;
ii. a second double-type plant;
iii. a second double-multi-type plant;
iv. a second single-type plant having the multi-petalled and globular shaped trait in its genetic background; or
v. a second single-type plant with no known multi-petalled and globular shaped trait in its genetic background; or
vi. a third multi-petalled-type; and
d. selecting multi-petalled-type and globular shaped flowering progeny.

15. The method according to claim 14, wherein said first, second and third multi-petalled-type plants, or first and second single-type, semi-double-type, double-type or double-multi-type plants are the same or different cultivars.

16. A *Gerbera jamesonii* plant, with one or more multi-petalled-type and globular-shaped inflorescences with i) at least 320, to about 550, full or partial outer petals (ray florets) per inflorescence, ii) at least 150, to about 300, full or partial inner petals (disc florets) per inflorescence, and iii) at least 520, to about 800, full or partial total petals (total florets) per inflorescence, produced by one of the methods according to claims 6, 8, 10, 12 or 14.

17. The *Gerbera jamesonii* plant according to claim 16, wherein said plant is selected from the group consisting of the *Gerbera jamesonii* cultivars 'Tersunrutas', 'Tersram', 'Tersunev', 'Ternoom' and 'Terlob'.
